# EUROPEAN PATENT APPLICATION

(11) **EP 2 305 154 A1**
(43) Date of publication of application: **06.04.2011**
(21) Application number: 09016148.0
(22) Date of filing: 30.12.2009
(51) Int. Cl.: A61B 17/70

(54) **Rod holder and minimally invasive spine surgery system using the same**

(30) Priority: 30.09.2009 KR 20090093413
(71) Applicant: GS Medical Co., Ltd., 505-14 Gasan-dong Geumcheon-gu, Seoul 153-803 (KR)
(72) Inventor: Shin, Min Sik, 433 Apgujeong-dong Gangnam-gu Seoul (KR); Kim, Jin Soon, 17 Sincheon-dong Songpa-gu Seoul (KR)
(74) Representative: Schupfner, Georg

(57) **Abstract**

The present application relates to a rod holder (300) configured to operate in a two-stage loading manner, which allows a rod (200) to be easily received. Also, the present application relates to a minimally invasive system for spinal surgical operation, which allows a rod to be more accurately and stably received to a pedicle screw inserted into a vertebra by using the rod holder, a rod guide and a rod guide holder. The rod holder may control a gripped state of a rod with three stages: namely a first loading stage for moving back and fixing a loading unit to fixedly grip the rod; a second loading stage for rotatably gripping the rod; and a rod mounting stage for separating the rod from the rod holder. Also, the minimally invasive spine surgery system includes a pair of rod guides (500,600) connected to upper ends of a pair of pedicle screws (100) to form a moving path of the rod; a rod holder for gripping the rod; and a rod guide holder (800) for defining an insertion path of the rod holder to guide an insertion location of the rod.

## Description

### BACKGROUND OF THE INVENTION

### 1. Field of the Invention

The present invention relates to a rod holder, and more particularly, to a rod holder which is configured to operate in a two-stage loading manner to allow a rod to be easily mounted.

In addition, the present invention relates to a minimally invasive spine surgery system, and more particularly to, a minimally invasive spine surgery system, which allows a rod to be more accurately and stably mounted to a pedicle screw inserted into a vertebra by using the rod holder, a rod guide and a rod guide holder during a spinal surgical operation using a minimally invasive method, thereby minimizing a damage of tissues or nerves in a surgical site.

### 2. Description of the Related Art

In general, the spine is commonly composed of 24 vertebras (except a sacral vertebra) which are connected with each other by means of joints, called disks, interposed between them so as to support the spine and give a buffering act. In this way, the spine helps a person to keep his/her posture and also plays important roles such as giving a basis for motions and protecting the internal organs.

However, if a person maintains an abnormal posture for a long time, suffers from degenerative diseases caused by aging or receives a shock from the outside, the disk between joints of the vertebras may be damaged to cause a spine disk disease. This spine disk disease compresses nerves connected to various portions of a human body through the joints of the vertebras, which gives a pain to the person.

Thus, for disk patients, a disk near a damaged portion is removed so that the damaged portion of a spine is not pressed or compressed, and an artificial aid (e.g., cage) made of hollow metal or plastic material is filled with bone fragments and then inserted into a region from which the disk is removed. After that, a pedicle screw is inserted into and fixed to vertebra at upper and lower locations of the damaged disk, and a rod is then connected to the pedicle screw to allow a distance between the vertebras to be secured, thereby ensuring a normal bone fusion.

In such a spine surgical operation, the skin around the damaged vertebra is cut to remove the damaged disk or left as it was, and the pedicle screw is then inserted into vertebras above and below the damaged disk and the fixed. After that, a rod is connected thereto, and a bolt is then fastened to couple the disk to the pedicle screw.

However, this surgical operation causes a large invasive region which delays the recovery of the patient and results in bad satisfaction for the surgery due to a large wound, though it may reduce an operation time and ensure easier operation. Thus, in recent, minimally invasive surgery manners being capable of minimizing an invasive region during a spine surgery operation have been developed and used.

In the minimally invasive surgical operation, when a pedicle screw is inserted into vertebras above and below a damaged disk, in a state where a patient's skin is not cut, a canula is put into the patient's skin without cutting the patient's skin, the pedicle screw is then inserted into the canula and fixed to the vertebras, and a rod is subsequently inserted through the canula and a bolt is then inserted into and fixed to a head portion. The rod used herein has a bar shape, so that it is extremely difficult to insert the rod through the canula. Thus, in order to facilitate easy insertion of the rod, the shape of the rod is modified or various kinds of operation tools have been developed for the insertion of the rod.

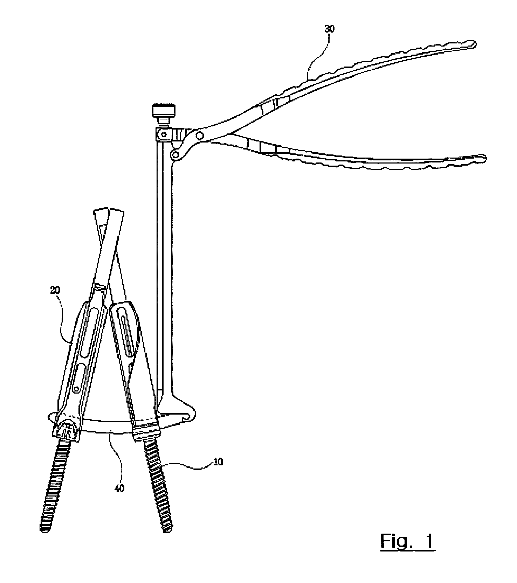

Fig. 1 is a schematic view showing a minimally invasive surgery operation tool disclosed in Korean Laid-open Patent Publication No. 2009-5316.

Referring to Fig. 1, a holding assembly 20 is connected to a pedicle screw 10 inserted into a vertebra to form a path through which a rod 40 can be inserted, and the rod 40 is then held at one end of the rod holder 30 and inserted through the path formed by the holding assembly 20.

However, in this method, while a surgeon grips the rod holder 30 and inserts the rod 40 into a surgical site, since the surgeon must grip a handle of the rod holder 30 and inset the rod holder into a patient's body at the same time, there is an inconvenience that the surgeon should pay careful attention to all two operations (that is, gripping and insertion). Due to the above, in a case where the gripping is released by a surgeon's carelessness before the rod is mounted, there is a danger that a surgical site should be enlarged to find the rod, so that the minimally invasive spine surgical method can be regarded as meaningless one.

In such surgical method, in the meantime, while a surgeon grips the rod holder 30 and inserts the rod 40 into a surgical site, if the holding assembly 20 connected to the pedicle screw 10 is shaken or the posture of the surgeon is unstable, it is difficult to control accurately an insertion direction of the rod 40, so that it is not easy to mount accurately the rod 40 to the pedicle screw 10. Accordingly, while the rod 40 is inserted, the rod 40 may damage or injure surrounding tissues or nerves, which may give a great pain to the patient after the surgery operation.

### SUMMARY OF THE INVENTION

Accordingly, an object of the present invention is to provide a minimally invasive spine surgery system which may solve the aforementioned problems in the prior art.

Another object of the present invention is to provide a rod holder which is easily manipulated and can make a rod be easily inserted thereto and be easily mounted thereto.

A further object of the present invention is to provide a minimally invasive spine surgery system which may allow a rod to be mounted to a pedicle screw more accurately and stably to minimize any damage of nerves or tissues around an operation portion when the rod is inserted.

A rod holder according to the present invention for gripping a rod, which connects a pair of pedicle screws inserted into and fixed to a vertebra with each other, to mount the rod to the pair of pedicle screws comprises a grip serving as a handle; a loading unit mounted to an upper portion of the grip in such a manner that the loading unit passes through the grip in a front and rear direction; a button unit mounted to the upper portion of the grip in such a manner that the button unit passes through the grip in a right and left direction, thereby cooperating with the loading unit; and an insert unit partially inserted into a human body and comprising a rod gripping portion for gripping the rod and a connection member for transmitting a forward or backward movement of the loading unit to the rod gripping portion. The rod holder is configured to adjust a gripped state of the rod through three sequential stage including a first loading stage for moving backward and fixing the loading unit to fixedly grip the rod; a second loading stage for partially moving the loading unit forward and fixing the loading unit to the button unit, thereby rotatably gripping the rod; and a rod mounting stage for completely moving the loading unit forward by operating the button unit so that the rod is separated from the rod holder.

A minimally invasive spine surgery system according to the present invention is to insert and fix a pair of pedicle screws into and to a vertebra and installing a rod for connecting the pair of pedicle screws with each other. The system comprises a pair of rod guides connected to upper ends of the pair of pedicle screws to form a moving path of the rod; a rod holder for gripping the rod; and a rod guide holder for guiding an insert position of the rod. Here, the rod guide holder includes a first fixing unit connected to upper ends of the pair of rod guides to keep a distance between the pair of rod guides constant; a guide connected to a center of the first fixing unit and having an arc shaped slit formed thereon; and a second fixing unit connected to the guide and moved circularly along the slit in a state where the rod holder is inserted therein, thereby guiding an insert position of the rod.

### BRIEF DESCRIPTION OF THE DRAWINGS

Fig. 1 is a schematic view showing a conventional minimally invasive spine surgery system;
Fig. 2 is a schematic view showing a minimally invasive spine surgery system according to the present invention;
Fig. 3 is a perspective view of a pedicle screw;
Fig. 4 is a schematic view showing a rod;
Fig. 5 is a side sectional view showing an overall configuration of a rod holder according to the present invention;
Fig. 6 is an exploded view showing an insert unit of the rod holder of Fig. 5;
Fig. 7 is a schematic view of a rod gripping portion of the insert unit of Fig. 6;
Fig. 8 is an exploded view of a loading unit of the rod holder of Fig. 5;
Fig. 9 is a schematic view showing a connection unit and a cover unit mounted to a grip;
Fig. 10 is a schematic view showing a two-stage loading adjusting member of the loading unit of Fig. 8;
Fig. 11 is a schematic view showing a button unit and a grip to which the button unit is mounted, provided in the rod holder of Fig. 5;
Figs. 12a to 12c are schematic views showing the rod holder in a first loading stage;
Figs. 13a to 13c are schematic views showing the rod holder in a second loading stage;
Figs. 14a to 14c are schematic views showing the rod holder in a rod mounting stage;
Fig. 15 is a schematic side sectional view of a rod holder according to another embodiment and an exploded and enlarged view of a locking unit;
Figs. 16a and 16b are a rear sectional view and a schematic perspective view of a rod holder according to another embodiment in a case where the locking unit is in a locked state and a rear sectional view and a schematic perspective view of the rod holder according to another embodiment in a case where the locking unit is in an unlocked state;
Fig. 17 is a schematic view showing a first embodiment of the rod guide;
Fig. 18 is a schematic view showing a second embodiment of the rod guide;
Fig. 19 is a sectional view of a rod guide of Fig. 18;
Fig. 20 is a view showing an inner body of the rod guide of Fig. 18;
Fig. 21 is a view showing an outer sleeve of the rod guide of Fig. 18;
Fig. 22 is a view showing a rod pusher of Fig. 2;
Fig. 23 is a schematic side view of a sleeve of a rod guide separator;
Fig. 24 is a partial enlarged view and schematic view of a circled portion A of Fig. 23;
Fig. 25 is a schematic side view of an insert bar of the rod guide separator;
Fig. 26 is a schematic view showing an operation mechanism of the rod guide separator;
Fig. 27 is a perspective view of the rod guide holder of Fig. 2;
Figs. 28a and 28b are a side view and a plane view of the rod guide holder of Fig. 27;
Fig. 29 is a view showing a state where the rod guide holder of Fig. 27 is installed;
Fig. 30 is a view showing a gap adjuster of Fig. 2;
Fig. 31 is a schematic perspective view of a gap adjuster according to another embodiment;
Fig. 32 is a schematic view showing an operation mechanism of the gap adjuster of Fig. 31; and
Fig. 33 is a schematic view showing that the gap adjuster of Fig. 31 adjusts a gap between pedicle screws.

### DESCRIPTION OF THE PREFERRED EMBODIMENT

Hereinafter, a preferred embodiment of the present invention will be explained in detail with reference to the accompanying drawings. For reference, when illustrating the present invention, any related known function or configuration may not be explained in detail herein if such function or configuration has a possibility to make the present invention unclear.

Prior to explaining the embodiments of the present invention, in the below description, a direction toward the spine to be surgically operated is defined as a 'front' direction, and a direction toward a surgeon is defined as a 'rear' direction based on a location of a rod holder. Similarly, a movement toward the spine is defined as a 'forward' movement, and a movement toward the surgeon is defined as a 'backward' movement.

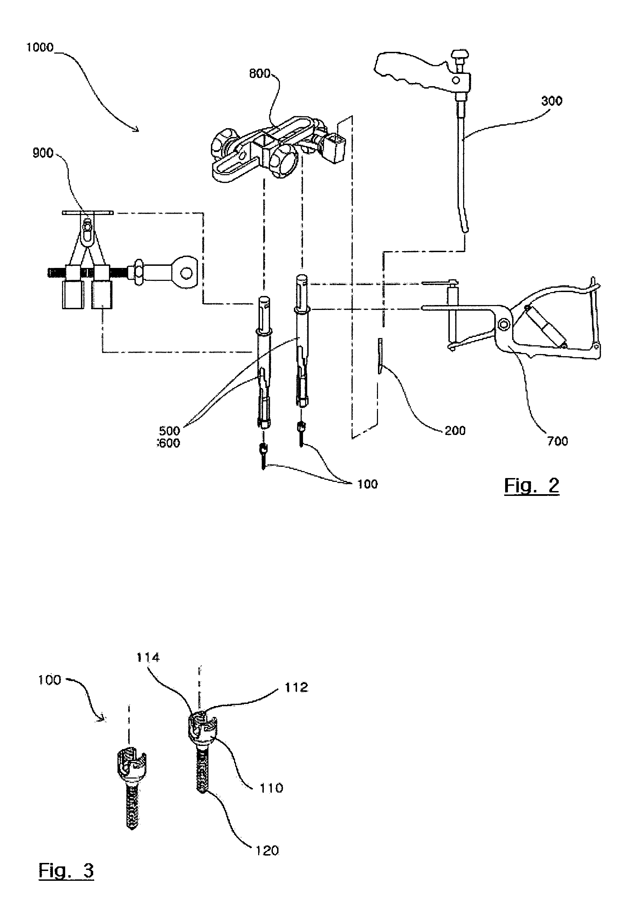

Fig. 2 shows a schematic view illustrating an entire configuration and connection relations among all components of a minimally invasive spine surgery system according to the present invention. As described above, the minimally invasive spine surgery system 1000 is a kind of operation system in which a pair of pedicle screws 100 are inserted into and fixed to the vertebra and a rod 200 for connecting the pair of pedicle screws 100 with each other is installed. Referring to Fig. 2, the minimally invasive spine surgery system 1000 of the present invention comprises a pair of pedicle screws 100 inserted into and fixed to the pedicle, a rod 200 for connecting the pair of pedicle screws 100 with each other, a rod holder 300 for gripping the rod 200, a pair of rod guides 500, 600 connected to upper ends of the pair of pedicle screws 100 to form a moving path of the rod 200, and a rod guide holder 800 connected to upper ends of the pair of rod guides 500, 600 to define a path through which the rod holder 300 gripping the rod is inserted into a patent's body.

Also, the minimally invasive spine surgical system 1000 may comprise a rod pusher 700 selectively for mounting closely the rod 200 to a pedicle screw and a gap adjuster 900 connected to sides of the rod guides 500, 600 to adjust a gap between the pedicle screws.

Fig. 3 is a view showing a configuration of the pedicle screw 100. As shown in Fig. 3, the pedicle screw 100 has a U shaped receiving groove 114 with a predetermined depth for receiving the rod 200, and the pedicle screw 100 includes a head portion 110 having a female thread 112 formed along an inner circumference surface of the receiving groove 114 and a screw portion 120 connected to a lower portion of the head portion 110 to be inserted into the vertebra. Here, although the pedicle screw 100 in which the head portion 110 and the screw portion 120 extend and are integrated with each other is illustrated herein, the pedicle screw 100 in which the head portion 110 is pivotally connected to one end of the screw portion 120 may be utilized. Preferably, a symmetrical groove is formed in an outer circumference surface of the head portion 110 to enable the rod guides 500, 600 explained later to grip the head portion 110.

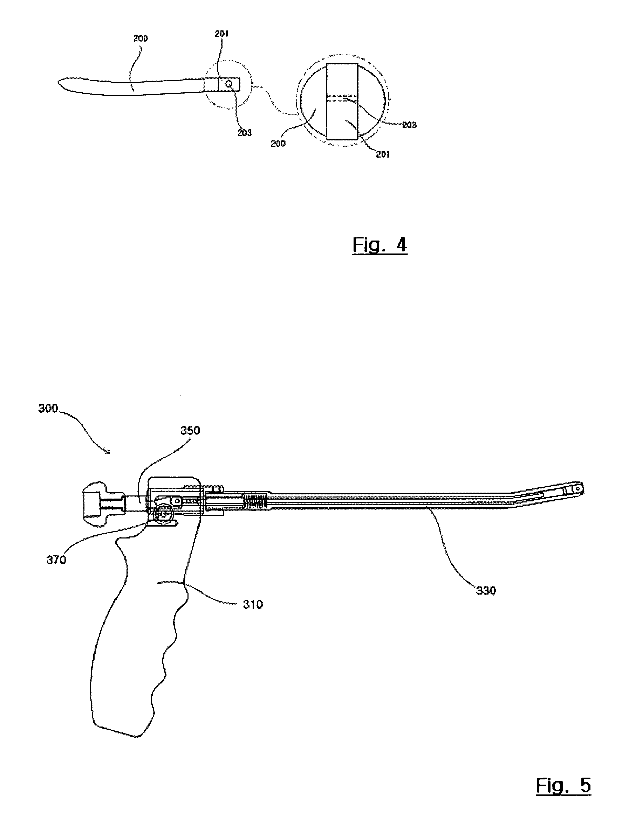

Fig. 4 is a view showing the rod 200 constituting the minimally invasive spine surgery system 1000 according to the present invention. As shown in Fig. 4, the rod 200 has a curved-bar shape, and one end of the rod 200 has a streamlined shape to facilitate easier insertion into a surgical site and an insertion portion 201 having a rectangular section extends from the other end of the rod 200. The insertion portion 201 is inserted into the rod holder 300, and a through hole 203 or a pair of fixing grooves to be gripped by the rod holder 300 may be formed on the insertion portion 201. As explained below, since the insertion portion 201 has a rectangular shape, if the insertion portion 201 is inserted into the rod holder 300 explained later, flat upper and lower surfaces of the insertion portion 201 are fixed and supported in the rod holder 300, so that the rod 200 cannot be rotated upward or downward.

Figs. 5 to 14 are schematic views showing a structure and an operation mechanism of the rod holder 300 of the minimally invasive spine surgery system 1000 according to the present invention.

Fig. 5 is a side sectional view showing an overall configuration of the rod holder 300 according to the present invention. The rod holder 300 is one of surgical tools of the minimally invasive spine surgery system 1000, which grips the rod 200 connecting a pair of pedicle screws 100 inserted into and fixed to the vertebra to mount the rod 200 to the pair of pedicle screws 100. Referring to Fig. 5, the rod holder 300 includes a grip 310 serving as a handle; a loading unit 350 passing through the grip 310 in a forward and backward direction and mounted to an upper portion of the grip 310; a button unit 370 passing through the grip 310 in a right and left direction and mounted to an upper portion of the grip 310 to allow the loading unit 350 to cooperate therewith;
and an insert unit 330 located at a front of the loading unit 350 and inserted partially into a human body while gripping the rod 200. The rod holder 300 is operated in three stages, namely a first stage in which a surgeon grips securely the rod 200 so as to easily insert the rod 200 into a patient's body, a second stage in which the surgeon mounts safely the rod 200 to the head portion of the pedicle screw 100, and a mounting stage in which the grip of the rod 200 is released. This operating mechanism will be explained in more detail after each component of the rod holder 300 is described.

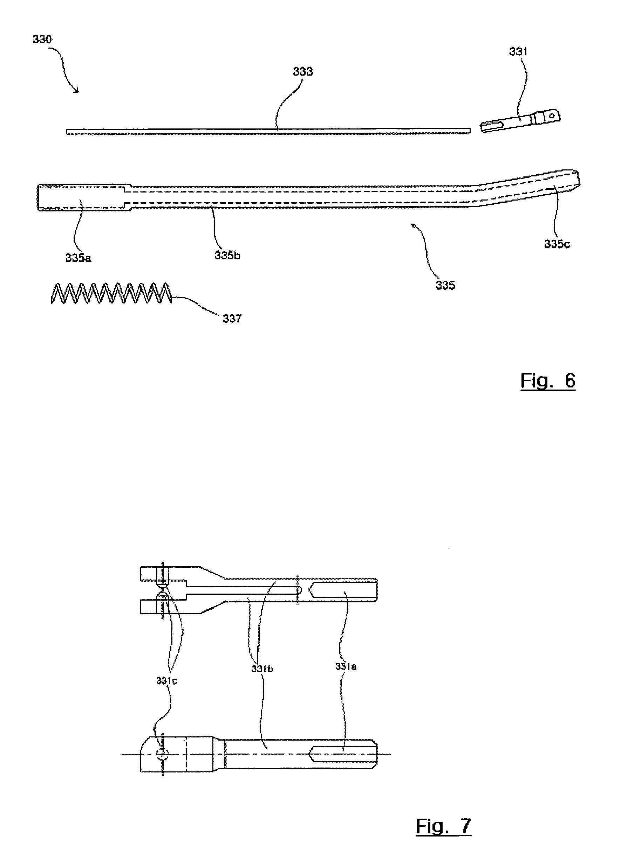

Referring to Fig. 6, the insert unit 330 includes a rod gripping portion 331 for gripping the rod 200, a connection member 333 fixedly connected to a rear end 331a of the rod gripping portion 331 and a hollow insert body 335 surrounding the rod gripping portion 331 or the connection member 333.

The connection member 333 is fixedly connected to the rear end 331a of the rod gripping portion 331 by means of welding or the like. Preferably, the connection member 333 may be formed integrally with the rod gripping portion 331. The connection member 333 is configured to transmit a forward or backward movement of the loading unit 350. In other words, the connection member 333 is configured to enable the rod gripping portion 331 to be moved by a distance corresponding substantially to a moving distance of the loading unit 350 when the loading unit 350 is moved forward or backward. For this purpose, in particular, it is preferable that the connection member 333 is made of wire, cable, bar shaped member or the similar mean, each of which having a rigidity for preventing the connection member from being buckling when the connection member 333 is moved forward. As explained below, the rear end of the connection member 333 is fixedly connected to an intermediate member 351 of the loading unit 350.

As shown in Figs. 6 and 7, the rod gripping portion 331 includes a rear end 331a fixedly connected to the front end of the connection member 333 by means of welding or the like and two arms 331b extending into two parts from the rear end 331a toward the front. Two arms 331b are divided into a Y shape to be elastically compressed inward, so that the gripping state of the rod 200 (or, a gripping state or a grip-releasing state) may be adjusted depending on whether two arms 331b are compressed inward. Protrusions 331c are formed on facing inner surface of the ends of the two arms 331b, and the protrusions 331c have a shape complementing to that of a pair of fixing grooves or through holes 203 of the rod 200. Thus, when the two arms 331b are compressed inward, the rod 200 may be firmly gripped.

As shown in Fig. 6, the insert body 335 receives the rod gripping portion 331 or the connection member 333 (see Fig. 5), and a front end portion of the insert body 335 is directly inserted into a patient's body. The insert body 335 is located at the front of the loading unit 350 and connected to the connection unit 311 (explained later) through a fitting manner or a fixedly-coupling manner such as welding.

The insert body 335 includes a rear end 335a for receiving the intermediate member 351 of the loading unit 350 explained later, a connection member-guide unit 335b for defining a path of the connection member 333 and a rod gripping portion receiver 335c extending from the front end of the connection member-guide unit 335b.

Preferably, the connection member guide unit 335b of the insert body 335 has a plurality of slot shaped openings. Due to the plurality of slot-like openings, it is possible to clean easily the insert body 335 after a surgical operation.

The rear end 335a may receive an elastic means such as a spring 337 to make the loading unit 350 be elastically moved forward. Also, the rear end 335a forms a path through which the loading unit 350 may be elastically moved forward or backward.

Preferably, the connection member guide unit 335b may make a predetermined angle with the rod gripping portion receiver 335c to place the rod 200 easily at the pedicle screw 100.

The interior of the rod gripping portion receiver 335c is configured to press two arms 33ab of the rod gripping portion 331 when the rod gripping portion 331 is inserted into the rod gripping portion receiver 335c by the connection member 333, to enable the two arms 331b of the rod gripping portion 331 to be shrunken inward. Preferably, the rod gripping portion receiver 335c may be configured to receive the rod gripping portion 331 entirely,

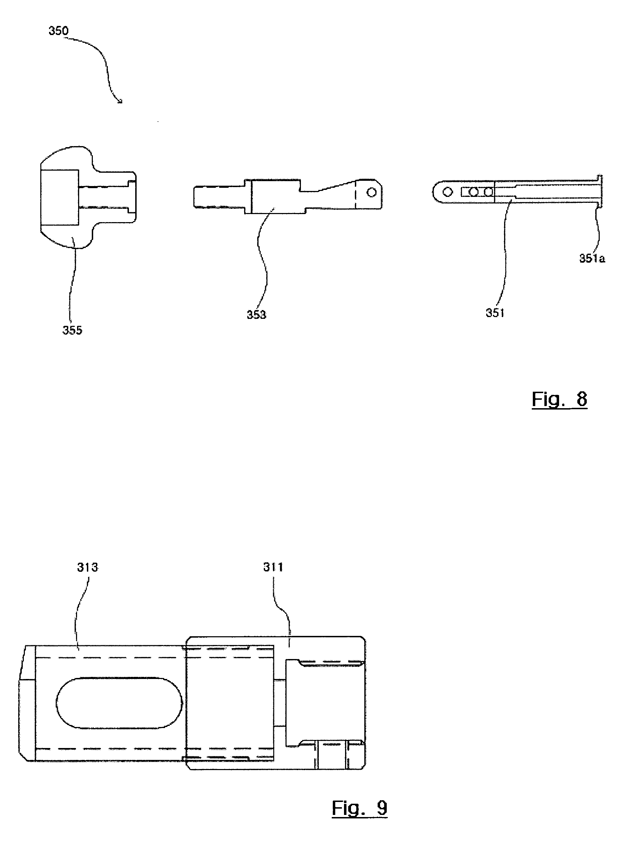

Fig. 8 is a schematic view showing a configuration of the loading unit 350. As shown in Fig. 8, the loading unit 350 includes an intermediate member 351 fixedly connected to a rear end of the connection member 333, a two-stage loading adjustment member 353 connected to a rear end of the intermediate member 351 and a loading handle 355 fixedly connected to a rear end of the two-stage loading adjustment member 353.

Referring to Fig. 8, the intermediate member 351 is partially or entirely received in the rear end 335a of the insert body 335 and moved forward or backward in the rear end 335a. A protrusive rim 351a is formed on a front end of the intermediate member 351 (in this reason, as shown in Fig. 8, overall the intermediate member 351 has a T shape), and an elastic member (e.g., spring 337) to be inserted into the rear end 335a of the insert body 335 is provided at the protrusive rim 351a, so that the intermediate member 351 is elastically moved forward or backward in the rear end 335a of the insert body 335. Preferably, a restoring force of the spring 337 is utilized for moving the intermediate member 351 or the entire loading unit 350 forwards.

As shown in Figs. 5 and 9, it is preferable that a connection unit 311 defining a path of the intermediate member 351 and connected to the insert unit 330 is installed at a front of the upper side of the grip 310 through which the loading unit 350 passes.

Also, a cover unit 313 having a stop formed thereon is connected to the rear end of the connection member 311, the stop of the cover unit being configured such that a first catching step 353d which will be described later can be fixed to the stop of the cover unit in a first loading stage.

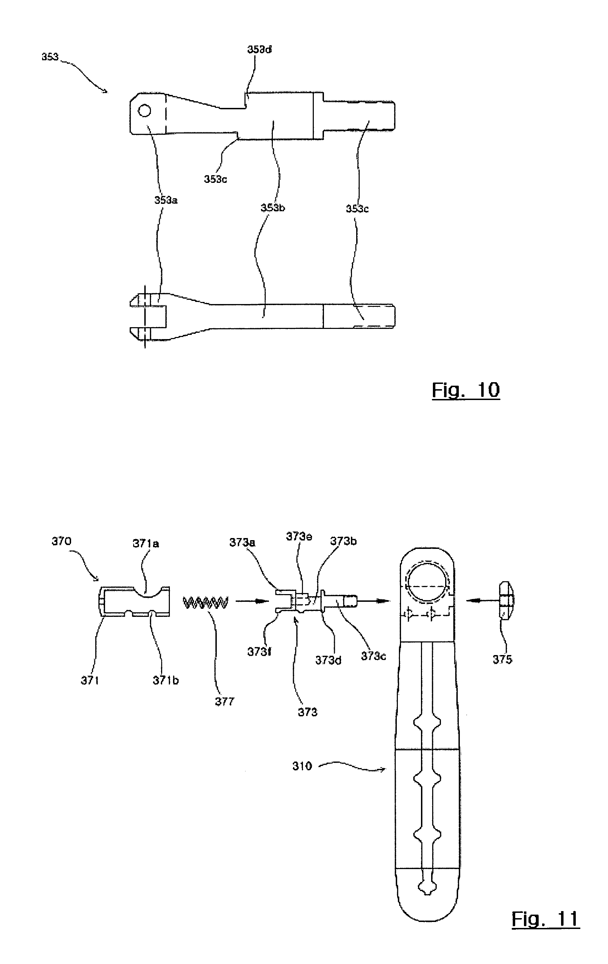

As shown in Fig. 10, the two-stage loading adjustment member 353 includes a front end 353a fixedly connected to a rear end of the intermediate member 351, a centre portion 353b allowing the loading unit 350 to be loaded into two stages and a rear end 353c to which the loading handle 355 is fixedly installed.

A first catching step 353d forming the first loading stage is formed at the upper portion of the center portion 353b of the two-stage loading adjustment member 353, and a second catching step 353e arranged at the front of the first catching step 353d to form the second loading stage is formed at the lower portion of the two-stage loading adjustment member 353. A location of the first catching step 353d may be changed with that of the second catching step 353e. However, the following explanation is based on the first catching step 353d and the second catching step 353e arranged as above. Now, the operating mechanism of the rod holder 300, which will be explained in more detail later, will be explained. The first catching step 353d is fixed at the stop formed at the rear end of the cover unit 313 to set the first loading stage, and the catching step 353e is fixed to a second loading stage adjuster 373 of the button unit 370 (explained later) to set the second loading stage. In other words, the two-stage loading adjustment member 353 may adjust the entire loading process of the loading unit 350 through a stage in which the first catching step 353d and the second catching step 353e are fixed and a stage in which the two-stage loading adjustment member 353 is moved forward by a release of fixing of the first catching step 353d and the second catching step 353e.

As shown in Fig. 11, the button unit 370 is mounted such that the button unit passes through the grip 310 in a right and left direction, and the button unit 370 is located below the loading unit 350 and cooperates with the loading unit 350. The button unit 370 includes a housing 371 mounted in an upper portion of the grip 310, a second loading stage adjuster 373 received in the housing 371 and a button 375 secured to an end of the second loading stage adjuster 373.

One end of the housing 371 is closed, and the other end of the housing 371 is opened to allow the button 375 to be located in the housing. Also, an opening 371a is formed in a part of the upper portion of the housing 371 to enable the two-stage loading adjustment member 353 of the loading unit 350 to pass through the upper portion of the housing 371 and be moved forward or backward. In addition, a groove 371b having a shape complementing to the protrusion formed on the grip 310 is formed on a lower portion of the housing 371 in order to prevent the housing 371 from being separated due to the force occurring when the button 375 is pressed (that is, to fix the housing 371 to the grip 310).

The second loading stage adjuster 373 includes a hollow portion 373a receiving a spring 377 by which the second loading stage adjuster can elastically reciprocate, a center portion 373b extending from the hollow portion 373a, and a button fixing unit 373c extending from the center portion 373b and to which the button 375 is fixed. An anti-separation protrusion 373d extends toward a rim between the center portion 373b and the button fixing unit 373c so as to prevent the second loading stage adjuster 373 from being separated outward.

Preferably, a step portion 373e is formed at an upper portion of the center portion 373b, so that a fixing or a forward movement of the second catching step 353e formed on the lower portion of the two-stage loading adjustment member 353 of the loading unit 350 can be adjusted. In other words, when the second loading stage adjuster 373 is pushed to the right (based on Fig. 11) due to the tensile restoring force of the spring 377, the step portion 373e is stopped at the second catching step 353e of the second loading stage adjuster 373 to set a second loading stage, and when the button unit 370 is pressed, an engagement between the step portion 373e and the second catching step 353e is released so that the two-stage loading adjustment member 353 is moved forward to achieve a rod mounting stage in which the gripping of the rod 200 is released. Even at this time, although the tensile restoring force of the spring 377 is still exerted to push the second loading stage adjuster 373 to the right (based on Fig. 11), the right side of the step portion 373e (based on Fig. 11) is contacted and engaged with a side surface of the two-stage loading adjustment member 353, so that it is prevented that the second loading stage adjuster 373 is restored to a location before the button 375 is pressed. In addition, a plurality of protrusions 373f, which are contacted and engaged with the protrusions formed on the inside of the housing 371 in correspondence with the grooves formed in the housing 371, are formed on the lower portion of the second loading stage adjuster 373 to define a reciprocating range in the second loading stage.

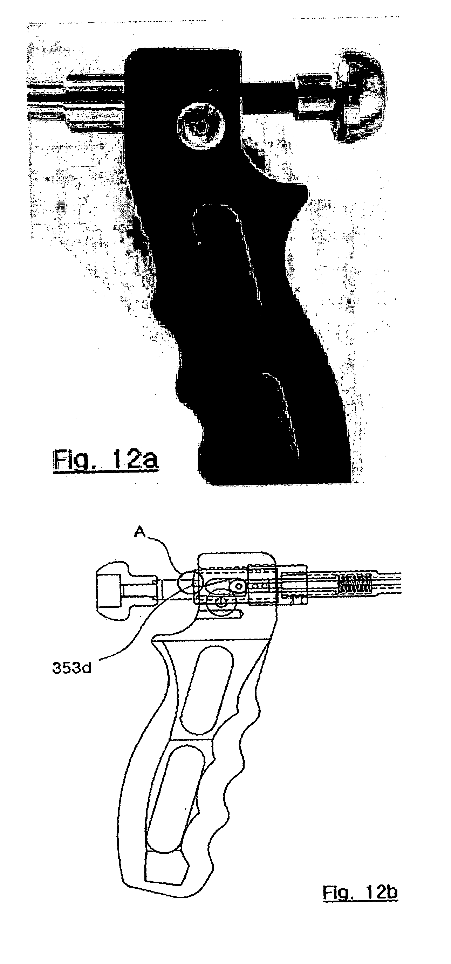

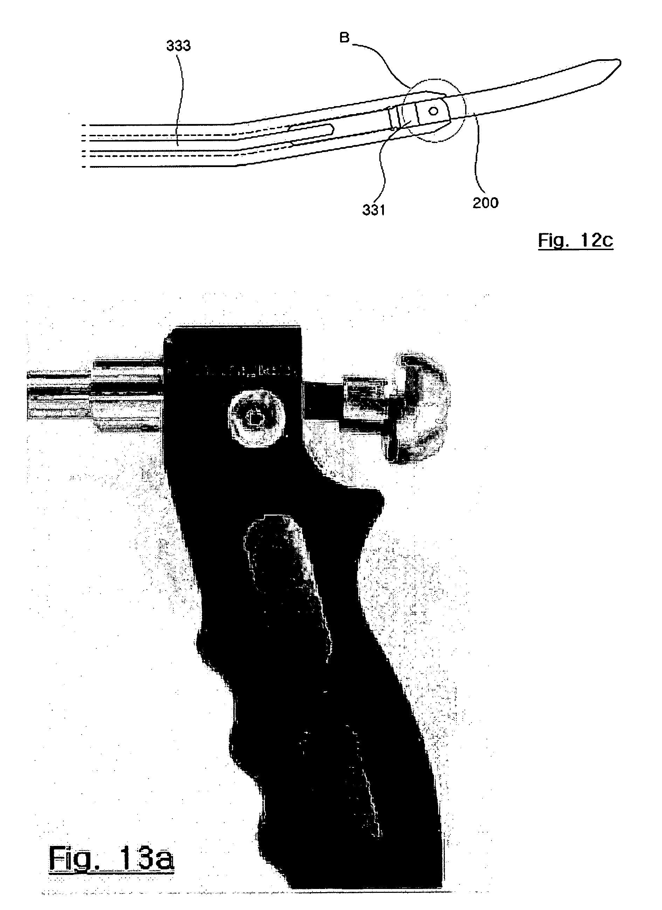

Hereinafter, an operating mechanism of the rod holder 300 according to the present invention is described with reference to Figs. 12 to 14.

Seeing the operating mechanism of the rod holder 300 in general, the rod holder 300 is operated to control a grip state of the rod 200 in a three-stage manner, including a first loading stage in which the loading unit 350 is moved backward to grip fixedly the rod 200; a second loading stage in which the loading unit 350 is partially moved 350 forward from the first loading stage and is then fixed to the button unit 370 to rotatably grip the rod 200; and a rod mounting stage in which the button unit 370 is operated from the second loading stage to completely move the loading unit 350 forward such that the rod 200 is separated from the rod holder 300.

At this time, the rod 200 in a gripped state, the rod gripping portion 331 gripping the rod 200, the connection member 333 fixedly connected to the rear end of the rod gripping portion 331, the intermediate member 351 fixedly connected to the rear end of the connection member 333, the two-stage loading adjustment member 353 fixedly connected to the rear end of the intermediate member 351, and the loading handle 355 fixedly connected to the rear end 353c of the two-stage loading adjustment member 353 (hereinafter, an assembly consisting of these components are called as 'a movable assembly') are integrally moved forward or backward. Preferably, the fixed connection among the components of the movable assembly may be achieved by welding, hole or pin connection or similar means.

Here, the force causing the forward movement of the movable assembly is a compressive restoring force of an elastic member (e.g., sprig 337) received in the rear end portion 335a of the insert body 335 and having one end fixed to the protrusive rim 351a of the intermediate member 351 of the loading unit 350 and the other end fixed in the rear portion 335a of the insert body 335, and the force causing the backward movement of the moving assembly is an external force exerted by a surgeon who pulls the loading handle 355 backward.

Figs. 12a to 12c illustrate the first loading stage. Fig. 12a is a side view of the rear portion of the rod holder 300 in the first loading stage, Fig. 12b is a side sectional view of the rear portion of the rod holder 300 in the first loading stage, and Fig. 12c is a side sectional view of the front portion of the rod holder 300 in the first loading stage.

As shown in Fig. 12a, in the first loading stage, a surgeon holds the grip 310 with one hand and holds the loading handle 355 with the other hand, and the surgeon pulls backward the loading handle 355 until the first catching step 353d of the loading unit 350 and the stop of the cover unit 313 are located on a straight line and then slightly lifts up the loading handle 355 to fix the two-stage loading adjustment member 353.

Even while the surgeon pulls the loading handle backward for setting the first loading stage, the movable assembly tends to move forward due to a compressive restoring force of the elastic member 337 installed to the protrusive rim 351a of the intermediate member 351. As shown in Fig. 12b, the loading handle 355 is slightly lifted up such that the first catching step 353d formed at the upper portion of the two-stage loading adjustment member 353 is engaged with the stop provided at the rear end of the cover unit 313 mounted to the upper end of the grip 310. As a result, a forward movement of the movable assembly may be controlled. A circled portion A of Fig. 12b illustrates the state that the first catching step 353d of the two-stage loading adjustment member 353 is engaged with the stop of the cover unit 313.

When the movable assembly is moved backward until the first catching step 353d of the loading unit 350 and the stop of the cover unit 313 are located on a straight line, the rod gripping portion 331 is moved backward by a distance as much as a backward moving distance of the movable assembly due to the connection member 333. At this time, the rod gripping portion 331 is completely inserted into the rod gripping portion receiver 335c of the insert body 335, and in this inserting process, the end or inner shape of the rod gripping portion receiver 335c compresses two arms 331b of the rod gripping portion 331 to shrink the two arms 331b elastically inwards, so that the protrusions formed on the two arms 331b grip firmly the fixing groove or the through hole 203 of the rod 200. In this state, as shown in Fig. 12c, the insertion portion 201 of the rod 200 on which the fixing groove or the through hole 203 are formed, which is gripped by the rod gripping portion 331, is inserted into the rod gripping portion receiver 335c, and the end of the rod gripping portion receiver 335c becomes substantially identical to the rear end of the rod in shape and height, thereby supporting the rod 200 in both upper and lower directions and thus enabling the rod 200 not to be rotated. In other words, the insertion portion 201 has a rectangular sectional shape (see Fig. 4), and so, in case where the insertion portion 201 is inserted into the rod holder 300 explained later, the flat upper and lower surfaces of the insertion portion 201 are fixed and supported by the upper and lower surfaces in the rod gripping portion receiver 335c, as a result, the rod 200 cannot be rotated upward or downward. A circled portion B of Fig. 12c illustrates a state that the rear end of the rod 200 is inserted into the rod gripping portion receiver 335c and thus the rod 200 can not be rotated.

In the first loading stage, the movable assembly is integrally moved forward or backward, so that a distance from a position at which the loading process is initiated to a position at which the first catching step 353d of the loading unit 350 and the stop of the cover unit 313 are located together on a straight line is substantially identical to a distance from a position at which the loading process is begun to a position at which the rod gripping portion 331 connected to the connection member 333 is completely inserted into the rod gripping portion receiver 335c (see Fig. 12c) by pulling backward the rod gripping portion.

In a state that the first loading stage is completed, the rod 200 is firmly gripped by the rod gripping portion 331 and can not be rotated, so that a surgeon may not worry about whether the rod 200 is fixed or rotated, so that the surgeon may insert the rod safely and easily into a patient's body while holding the grip 310 of the rod holder 300.

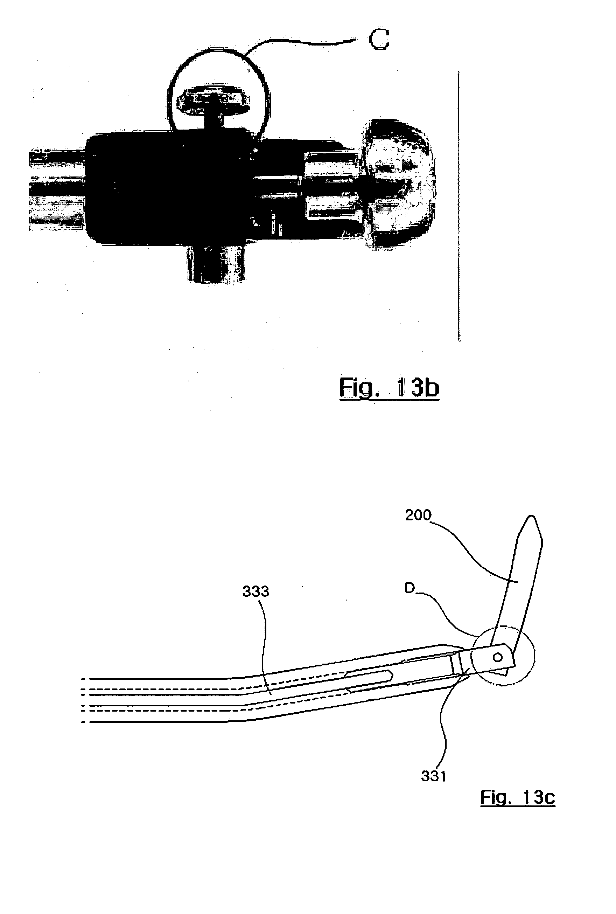

Figs. 13a to 13c illustrate the second loading stage. Fig. 13a is a side view of a rear portion of the rod holder 300 in the second loading stage, Fig. 13b is a plane view of the rear portion of the rod holder 300 in the second loading stage, and Fig. 13c is a side sectional view of a front portion of the rod holder 300 in the second loading stage.

As shown in Fig. 13a, in the second loading stage, if a surgeon holds the grip 310 with one hand and slightly moves down the loading handle 355 fixed in the first loading stage with the other hand, the movable assembly is partially moved forward by the elastic member installed to the intermediate member 351, and during the forward movement, the second catching step 353e formed at the two-stage loading adjustment member 353 is caught to the step portion 373e of the second loading stage adjuster 373.

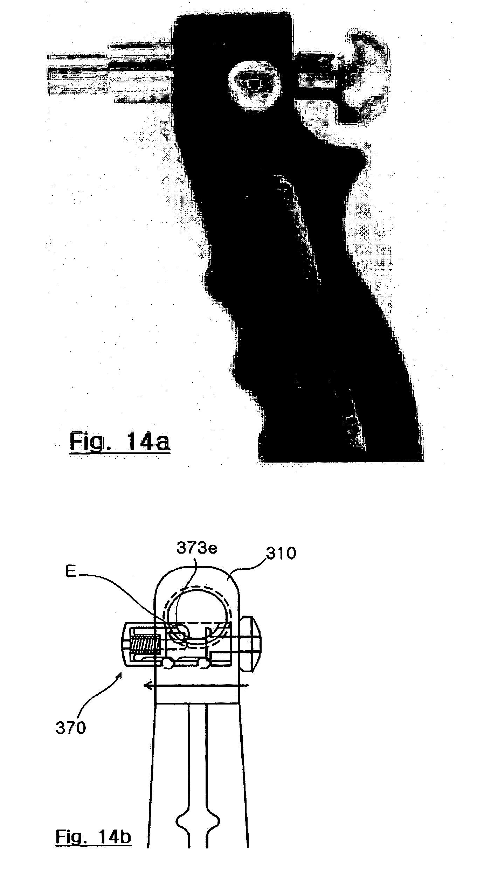

In other words, as shown in Fig. 13b, since the button 375 is not yet pressed in the second loading stage, the button 375 is located spaced apart from the grip 310 by a predetermined distance by means of the spring received in the hollow portion 373a of the second loading stage adjuster 373 (see a circled portion C of Fig. 13b), and the step portion 373e is located to block a forward movement path of the second catching step 353e of the two-stage loading adjustment member 353 (see a circled portion E of Fig. 14b).

As shown in Fig. 13c, due to the partial forward movement of the movable assembly, the rod gripping portion 331 is partially moved forward out of the rod gripping portion receiver 335c, so that a gripping state of the rod 200 is maintained, but the insertion portion 201 of the rod is not supported by the rod gripping portion receiver 335c, so that the rod 200 can be rotated in upper and lower directions (see a circled portion D of Fig. 13c). At this time, since the movable assembly is integrally moved forward or backward, a moving distance of the movable assembly from the first loading stage to a state where the second catching step 353e of the two-stage loading adjustment member 353 is engaged with the step portion 373e of the second loading stage adjuster 373 is substantially identical to a forward movement distance of the rod gripping portion 331 from the first loading stage to a state where the rod is rotatably gripped.

In a state that the second loading stage is completed, the rod 200 is rotatable. Thus, after the rod 200, explained later, passes through a cut portion of the rod guide, a surgeon may freely adjust an inserting angle of the rod, so that the surgeon may mount the rod to the head portion of the pedicle screw 100 easily and safely.

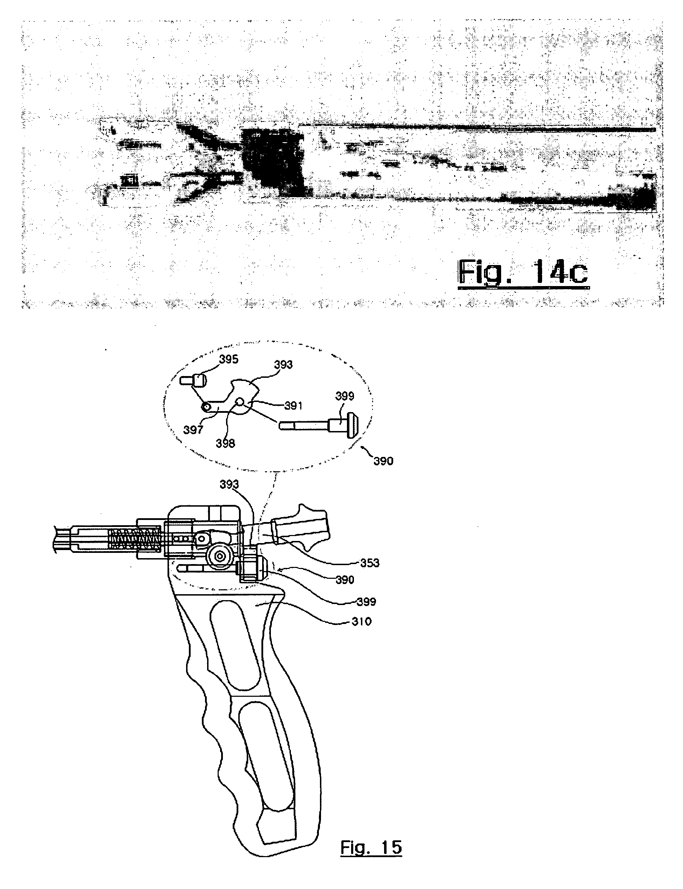

Figs. 14a to 14c illustrate a rod mounting stage. Fig. 14a is a side view of a rear portion of the rod holder 300 in the rod mounting stage, Fig. 14b is a sectional view of a rear portion of a button unit 370 and shows an operating location of the button unit 370 in the rod mounting stage, and Fig. 14c is a plane view of a front portion of the rod gripping portion 331 and shows a state of the rod gripping portion 331 in the rod mounting stage.

As shown in Figs. 14a and 14b, if the button 375 of the rod holder 300 in the second loading stage is pressed in an arrow direction of Fig. 14b, the step portion 373e of the second loading stage adjuster 373 is pushed in the arrow direction to open the forward movement path of the second catching step 353e of the two-stage loading adjustment member 353 (see a circled portion E of Fig. 14b), so that the movable assembly is completely moved forward by means of the elastic member (e.g., spring 337) installed to the intermediate member 351 to form the rod mounting stage.

As shown in Fig. 14c, in the rod mounting stage, two arms 331b of the rod gripping portion 331 are almost completely moved out of the rod gripping portion receiver 335c, so that the pressing force is eliminated. Accordingly, the two arms 331b are elastically restored into an original Y shape. In this reason, the protrusions 331c formed on the two arms 331b are released from the fixing groove or the through hole 203 of the rod to separate the rod 200 from the rod holder 300.

In order to grip the rod 200 again for a reloading, the fixing groove or the through hole 203 of the rod 200 are located at the protrusions 331c of the two arms 331b, and the loading handle 355 is then pulled to a location of the first loading stage. At this time, the second loading stage adjuster 373 is moved in the right direction again due to the spring 377 received in the hollow portion 373a thereof (based on Fig. 14b), so that the step portion 373e of the second loading stage adjuster 373 is re-located again to block the forward movement path of the second catching step 353e of the two-stage loading member.

As shown in Fig. 15, as another embodiment of the rod holder, the rod holder may further include a locking unit 390.

The locking unit 390 includes a circular body 391 located below the two-stage loading adjustment member 353 of the loading unit 350, a wing shaped locking projection 393 extending outward from one side of the circular body 391 in the radial direction to press the two-stage loading adjustment member 353 upwards, a bar shaped arm 397 extending outward in the radial direction from the other side of the circular body 391 and having a handle pin 395 mounted thereto, and a rotary central shaft 399 inserted into a through opening 398 formed at the center of the circular body 391 to fix the circular body 391 to the grip 310 rotatably. An angle between the arm 397 and a central line of the locking projection 393 is preferably 90° or above, more preferably 120°.

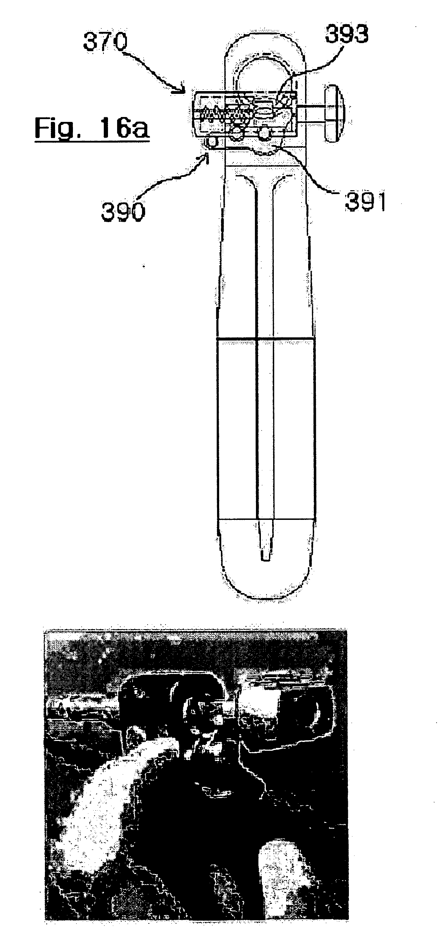

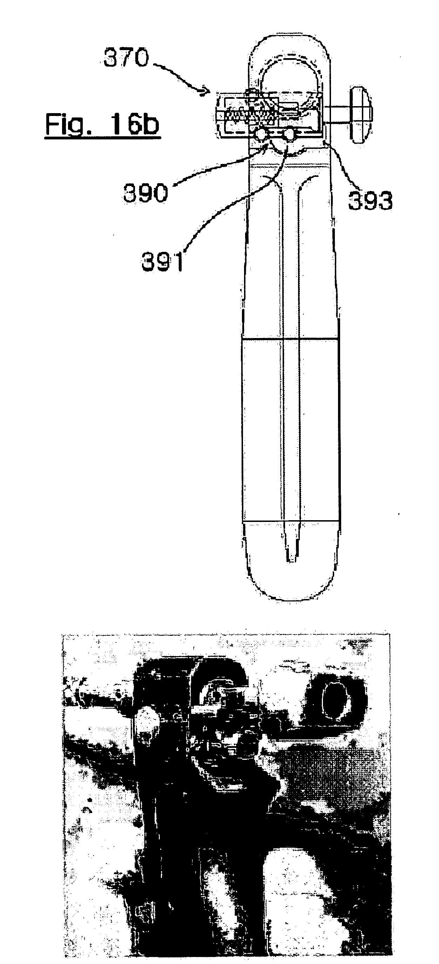

Seeing the operating mechanism of the locking unit 390, as shown in Figs. 16a and 16b, in case where the rod holder 300 is in the first loading stage, if a surgeon rotates the handle pin 395 mounted to the arm 397 in the counterclockwise direction, the locking projection 393 pushes up a lower portion of the two-stage loading adjustment member 353 (i.e., pressing it), and thus the two-stage loading adjustment member 353 is maintained in the first loading stage (a locked state). In this state, after the rod 200 is inserted into the pedicle screw 100 by means of the rod holder 300, once a surgeon rotates the handle pin 395 in the clockwise direction, the locking projection 393 is swerved from the lower portion of the two-stage loading adjustment member 353 so that a pressed state of the two-stage loading adjustment member 353 is released (releasing a locking state). Due to the above state, a surgeon may freely manipulate the rod holder 300 from the first loading stage to the second loading stage.

By using such locking unit 390, an unintentional downward movement of the loading handle 355 caused by a mistake of the surgeon can be prevented, so that it is possible to prevent the first loading stage from being converted into the second loading stage.

By employing the rod holder 300 configured as illustrated above, a surgeon may adjust easily a gripped state of the rod 200 through three stages including a gripped state in which the rod 200 is firmly fixed, a rotatably gripped state, and a grip released state by means of a simple manipulation such as pulling the loading handle 355 or pressing the button 375.

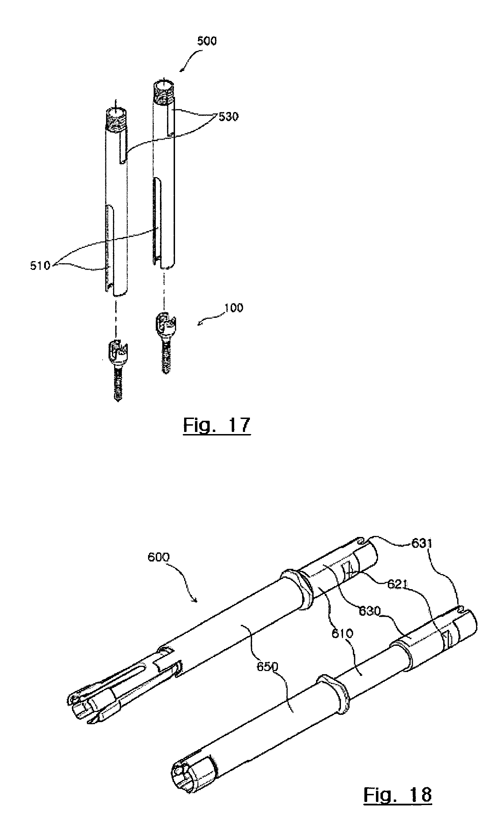

Figs. 17 to 21 show schematically rod guides 500, 600 constituting the minimally invasive spine surgery system 1000 according to the present invention. Fig. 17 shows the first embodiment of the rod guide 500, and Figs. 18 to 21 show the second embodiment of the rod guide 600.

As shown in Fig. 17, the rod guide 500 of the first embodiment has a hollow cylindrical shape and a pair of cuts 510 are formed at a lower end of the rod guide in opposite directions such that the rod 200 may be inserted therein. Also, a flat fixed surface 530 is formed at an upper end of the rod guide to be perpendicular to the cut 510 such that the rod guide may be inserted in and fixed to a rod guide holder 800 explained later. The fixed surface 530 makes it possible to prevent the rod guide 500 from being rotated in the rod guide holder 800 explained later, so that it is possible to fundamentally prevent locations of the cuts 510 serving as a moving path of the rod from being changed. In addition, a pair of plane grooves 521, which can be gripped by a rod pusher 700 explained later, may be symmetrically formed on an upper end surface of the rod guide 500, wherein the groove and the cut 510 are formed on a straight line.

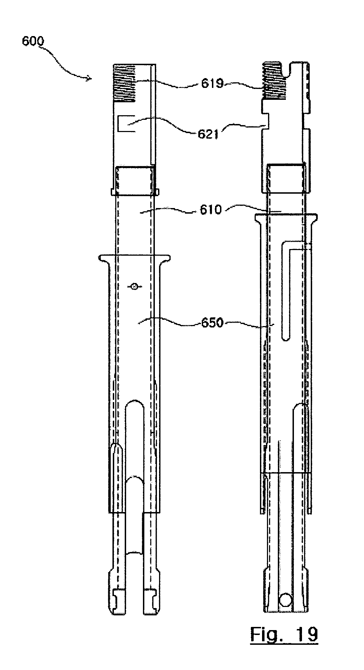

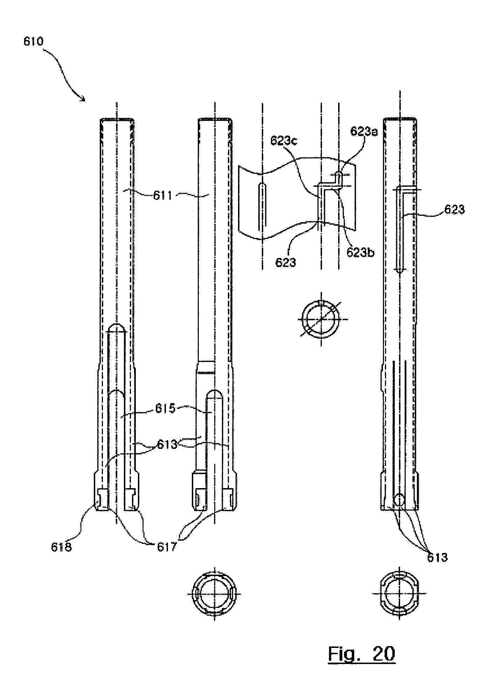

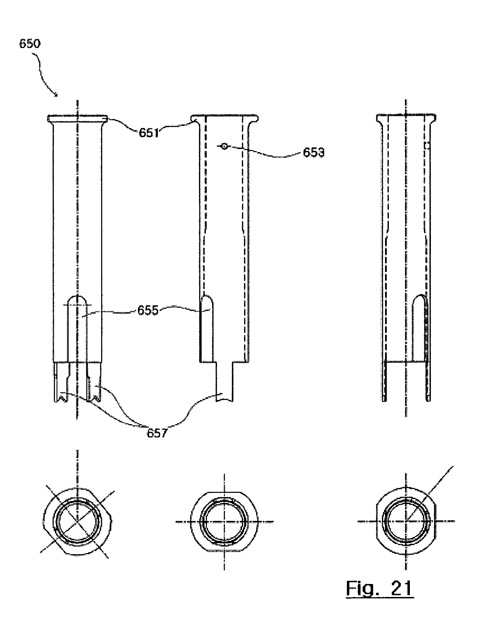

Figs. 18 to 21 show the rod guide 600 of the second embodiment. Fig. 18 is a perspective view showing the second embodiment of the rod guide 600, Fig. 19 is a sectional view showing the second embodiment of the rod guide 600, Fig. 20 is a sectional view of an inner body 610 of the rod guide 600 of the second embodiment, and Fig. 21 is a sectional view of an outer sleeve 650 of the rod guide 600 of the second embodiment.

As shown in Figs. 18 and 19, the rod guide 600 includes a hollow cylindrical inner body 610 for griping the pedicle screw 100 and an outer sleeve 650 surrounding the inner body 610 in an axial direction and slid in two stages along the inner body 610.

As shown in Fig. 20, the inner body 610 includes a hollow tube 611, a plurality of elastic arms 613 extending from the hollow tube 611 into a V shape, a pair of cuts 615 formed between the elastic arms 613 and facing to each other, and a gripping portion 617 formed at an end of the elastic arm 613 to grip the pedicle screw 100.

Preferably, as shown in Figs. 18 and 19, a thread may be formed on an upper end of the hollow tube 611 to allow a driver to be connected thereto. Also, a pair of plane grooves 621 to be gripped by the rod pusher 700 or the gap adjuster 900 (explained later) may be formed on the surface below the upper end of the hollow tube 611 on which the thread is formed. Here, the plane grooves 621 are symmetrically formed on a surface of the hollow tube rod, wherein the groove and the cut 615 are formed on a straight line. Preferably, the flat fixed surface 630 is formed on a side of the upper end of the hollow tube 611 to be perpendicular to the cut 615 such that the hollow tube can be inserted in and fixed to the rod guide holder 800 (see Fig. 18). As mentioned above, by using the fixed surface 630, it is possible to prevent the rod guide 600 from being rotated in the rod guide holder 800, so that it is possible to fundamentally prevent location of the cut 615 serving as a moving path of the rod 200 being changed. Preferably, in addition, the groove 631 guiding an installing direction of the rod guide separator 400 explained later may be formed at the upper end of the fixed surface 630.

A guide groove 623 defining a path of a guide pin 653 formed in the outer sleeve 650 explained later is formed at a side of the hollow tube 611 placed below the plane groove 621. The guide groove 623 includes, as shown in Fig. 20, a first vertical sliding guide 623a, a horizontal guide 623b formed perpendicular to the lower portion of the first vertical sliding guide 623a, and a second vertical sliding guide 623c formed perpendicular to the horizontal guide 623b in a lower direction of the inner body 610. The role of the guide groove 623 will be described in detail when an operating mechanism of the rod guide 600 is explained.

In order to enhance the elastic characteristics of the elastic arm 613, the elastic arm 613 is divided in a longitudinal direction into plural parts, and the elastic arm 613 is widen into a V shape so that the elastic arm can be shrunken inward or expansively restored. A gripping portion 617 having a protrusion 618 formed thereon for gripping the groove formed on an outer surface of the head portion of the pedicle screw 100 is provided at an end of the elastic arm 613. The gripping portion 617 grips the pedicle screw 100 as follows. In a state where the elastic arm 613 is shrunken inward, the pedicle screw 100 is gripped by the protrusion 618 of the gripping portion 617. Also, in a state where the elastic arm 613 is restored outward, the protrusion 618 releases the gripping of the pedicle screw 100. In other words, due to an elastic movement of the elastic arm 613, a gripping state of the pedicle screw 100 is adjusted.

The cut 615 serves as a path through which the rod 200 is inserted, and the cut 615 is formed in a longitudinal direction between the elastic arms 613 and in opposite directions to enable the rod to be inserted therein. Preferably, among four cuts formed on the pair of rod guides 600, the cut into which the rod 200 is inserted at first has a height causing an exposure out of the patient's skin so that the rod 200 may be inserted from the outside of the skin.
Due to the cut 615 having such a height, a surgeon may easily insert the rod 200 from the outside of a human body without expanding a surgical site such that the minimally invasive spine surgical operation can be performed pertinently.

As shown in Fig. 21, the outer sleeve 650 has a cylindrical hollow shape, and the inner body 610 is received in the outer sleeve 650. A protrusive rim 651 serving as a handle is formed at the upper end of the outer sleeve 650. Preferably, opposite surfaces of the protrusive rim 651 are bilateral symmetric and has flat shape, so that a surgeon may easily rotate the outer sleeve 650. The protrusive rim 651 also plays a role of a pressing unit against the rod pusher 700 explained later. A guide pin 653 corresponding to the guide groove 623 is formed in an upper inner surface of the outer sleeve 650 such that the outer sleeve 650 may be guided along the guide groove 623 formed in the inner body 610. Due to the guide pin 653 and the guide groove 623 as described above, the path of the outer sleeve 650 is defined as a vertical sliding path and a lateral rotating path with respect to the inner body 610.

On a lower end of the outer sleeve 650, a pair of cuts 655 extend in opposite directions, so that the rod 200 may be inserted into the rod guide 600 through the cuts 655. The cut 655 of the outer sleeve 650 may be located on a straight line with the cut 615 of the inner body 610 so as to enable the outer sleeve 650 to be rotated in a right or left direction to insert the rod into the rod guide 600. Among four cuts 615 formed on the pair of rod guides 600, the cut 655 into which the rod is inserted at first has a height causing an exposure out of the patient's skin so that the rod 200 may be inserted from the outside of the skin. Due to the cut 655 having such a height, a surgeon may insert easily the rod 200 from the outside of a human body without expanding a surgical site such that the minimally invasive spine surgical operation can be performed pertinently.

At least one rod pressing unit 657 is formed at a lower end of the outer sleeve 650 and extends in a longitudinal direction toward a downstream. Thus, when the rod 200 is located to the head portion of the pedicle screw 100 by means of the rod holder 300, the outer sleeve 650 can be moved in a lower direction to press the rod such that the rod is closely contacted to the head portion of the pedicle screw 100. It is preferable that the rod pressing unit 657is formed at a portion near the cut 655 such that a distance between a central axis of the rod pressing unit 657 and a central axis of the cut 655 is substantially identical to a length of the horizontal guide unit 623b of the rod guide groove 623. The end of the rod pressing unit 657 preferably has a concave shape corresponding to an outer shape of the rod in order to press surely the rod.

It is preferable that, among the pair of rod guides 600, the rod guide 600 to which the rod is inserted at first has only one rod pressing unit 657 formed on a side of the outer sleeve 650 located in an opposite direction to the insertion direction of the rod.

Since the rod holder 300 is arranged at the cut into which the rod 200 is inserted at first and the surgical operation is performed, the above configuration does not disturb the movement of the rod holder 300.

Due to the above rod pressing unit 657, the possibility that the rod 200 is suspended at a mid portion of the head portion of the pedicle screw 100 may be removed, so that it is possible to eliminate any inconvenience that the thread is not well fit when the set screw is fastened due to unstable mounting of the rod. In other words, the rod 200 may be closely contacted with the head portion of the pedicle screw 100 by the rod pressing unit 657, so that the rod 200 may be more securely mounted to the head portion of the pedicle screw 100.

In order to allow the rod pressing unit 657 to be in contact with rod 200 in a pressing manner, the rod pusher 700, explained later, is preferably used. In particular, it is preferable to employ the rod pusher 700 when the outer sleeve 650 is moved in a lower direction along the second vertical sliding guide unit 623c. Hereinafter, the rod pusher 700 is explained.

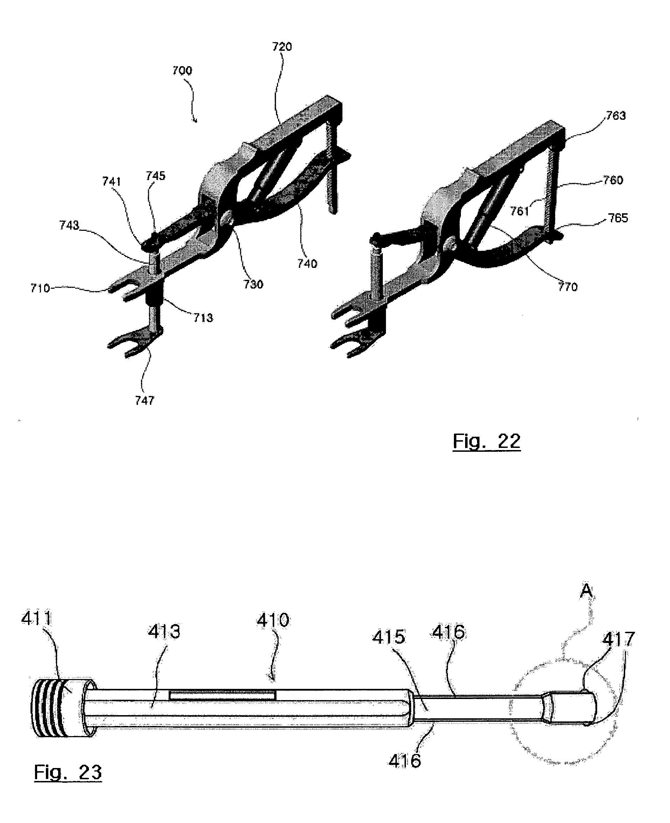

Fig. 22 is a schematic view showing configuration and operating mechanism of the rod pusher 700 which may constitute the minimally invasive spine surgery system 1000 according to the present invention. As shown in Fig. 22, the rod pusher 700 includes an upper handle 720 having a fixed gripping portion 710 for gripping a plane groove 621 formed in the inner body 610 of the rod guide 600 and a lower handle 740 connected to the upper handle 720 by a hinge 730. Preferably, the upper handle 720 and the lower handle 740 are connected by means of the hinge 730 in an X-like shape.

A pressing head 741 for generating a downward pressing force is formed at an end of the lower handle 740, and a shaft 743 to which a downward pressing force is exerted is connected to a lower portion of the pressing head 741 by a connection means such as a pin 745. Also, a movable gripping portion 747 is fixedly installed to a lower end of the shaft 743.

In addition, it is preferable that a sliding tube 713 is installed to a lower portion of the fixed gripping portion 710 of the upper handle 720 such that the shaft 743 may slide and a distance between the movable gripping portion 747 and the fixed gripping portion 710 is kept constant before the upper handle 720 and the lower handle 740 are pressed and gripped. The length of the sliding tube 743 is substantially identical to a distance between the protrusive rim 651 of the outer sleeve 650 and the plane groove 621 in case where the sliding tube is located above the second vertical sliding guide unit 623c so that the sliding tube 743 can make the rod pusher 700 be mounted easily to the rod guide 600 before the upper handle 720 and the lower handle 740 are pressed and gripped.

If the upper handle 720 and the lower handle 740 are pressed and gripped, the movable gripping portion 747 is in contact with the protrusive rim 651 of the outer sleeve 650 to press the outer sleeve 650 toward the pedicle screw while the fixed gripping portion 710 grips fixedly the plane groove 721. In other words, when the upper handle 720 and the lower handle 740 are pressed and gripped, a distance between the fixed gripping portion 710 and the movable gripping portion 747 is increased though a distance between the end of the upper handle 720 and the end of the lower handle 740 is decreased. Due to such a handle pressing process, the rod pressing unit 657 of the outer sleeve 650 presses the rod in a seesaw manner.

A distance adjusting means 760 for adjusting a moving distance of the movable gripping portion 747 is provided at ends of the upper handle 720 and the lower handle 740 located at a side opposite to the gripped portion of the rod guide 600. In other words, the bar shaped distance adjusting means 760 having toothed first projections 761 formed at one side thereof is further provided between the other end of the upper handle 720 and the other end of the lower handle 740 to adjust a moving distance of the movable gripping portion 747. An insertion hole 765 having a tooth shaped second projection formed at a portion contacted with the first projections is formed with a predetermined depth at the other end of the lower handle 740, so that the distance adjusting means 760 may be inserted thereto. One end of the distance adjusting means 760 is connected to the other end of the upper handle 720 by a hinge 763, and the other end of the distance adjusting means 760 is inserted into the insertion hole 765, so that one of concave portions between the first projections 761 is engaged with the second projection. Due to the above configuration of the distance adjusting means 760, if the upper handle 720 and the lower handle 740 are pressed, a moving distance of the movable gripping portion 747 may be adjusted step by step.

Also, it is preferable that the rod pusher 700 further includes an elastic member 770 provided between the upper handle 720 and the lower handle 740. Due to the above elastic member 770, when the fixed state of the distance adjusting means 760 is released, it is possible to recover the upper handle 720 and the lower handle 740 to a state where the handles are not yet pressed.

By using the rod pusher 700 as above, the outer sleeve 650 of the rod guide 600 of the second embodiment may be easily slid with a small power, and the rod pressing unit 657 may also compress the rod 200 more strongly with a small power.

Hereinafter, an operating mechanism of the rod guide 600 and the rod pusher 700 is explained.

First of all, the head portion 110 of the pedicle screw 100 is located at the gripping portion 617 of the inner body 610. At this time, the guide pin 653 of the outer sleeve 650 is located at the upper portion of the first vertical sliding guide unit 523a among the guide grooves 623 of the inner body 610.

Due to the above, since the lower end of the outer sleeve 650 is located in the hollow tube 611 of the inner body 610, any pressing force is not yet transferred to the elastic arm 613 of the inner body 610, so that the gripping portion 617 does not still grip the pedicle screw 100. Also, in this state, one pair of cuts 655 of the outer sleeve 650 are overlapped with one pair of cuts 615 of the inner body 610, so that the rod 200 can be inserted.

Then, until the guide pin 653 of the outer sleeve 650 is located below the first vertical sliding guide unit 623a among the guide grooves 623, the outer sleeve 650 is slid in a downward direction along the inner body 610. During this sliding process, the end of the outer sleeve 650 partially presses the plurality of elastic arms 613 elastically widen into a V shape to shrink inward the elastic arms 613, and the gripping portion 617 is also shrunken inwards according to the above inward shrinkage, so that the protrusion 618 formed on the gripping portion 617 firmly grips the groove of the head portion 110 of the pedicle screw in a spring collet manner. For reference, in this state, a screw driver is connected to the thread formed on the upper portion of the inner body 610 to fix the pedicle screw 100 gripped by the rod guide 600 to the vertebral pedicle. Also, even in this state, one pair of cuts 655 of the outer sleeve 650 are still overlapped with one pair of cuts 615 of the inner body 610, so that the rod may be inserted therein.

After that, the pedicle screw 100 is fixed to the vertebral pedicle, and the rod 200 is located to the head portion 110 of the pedicle screw 100 through the cuts 615, 655 of the rod guide 600 by using the rod holder 300. Then, the protrusive rim 623b of the outer sleeve 650 is gripped and then rotated in the left direction in Fig. 20 (namely, in a clockwise direction) with respect to the inner body 610 such that the guide pin 653 of the outer sleeve 650 is located at the left side (in Fig. 20) of the horizontal guide unit 623b of the guide groove 623. In this state, since the rod pressing unit 657 is formed near the cut 655 such that a distance between the central axis of the rod pressing unit 657 and the central axis of the cut 655 is substantially identical to a length of the horizontal guide unit 623b of the rod guide groove 623, the rod pressing unit 657 of the outer sleeve 650 and the cut 615 of the inner body 610 are located on a straight line as shown in Fig. 19 due to the above clockwise rotation.

In the state where the rod pressing unit 657 and the cut 615 of the inner body 610 are located on a straight line, the fixed gripping portion 710 of the rod pusher 700 is fit into the plane groove 621 of the inner body 610, and the movable gripping portion 747 is located on the protrusive rim 651 of the outer sleeve 650 such that the movable gripping portion 747 of the rod pusher 700 comes in contact with the protrusive rim 651 of the outer sleeve 650.

Then, the upper handle 720 and the lower handle 740 of the rod pusher 700 are pressed and gripped to move the movable gripping portion 747 downward (see the left side of Fig. 22). As shown in Fig. 22, due to the downward movement of the movable gripping portion 747, the outer sleeve 650 is also pressed downward, so that the rod pressing unit 657 of the outer sleeve 650 presses the rod 200 to be closely contacted with the head portion 110 of the pedicle screw 100. At this time, the guide pin 653 of the outer sleeve 650 is moved up to a lower position along the second vertical sliding guide unit 623c.

After the rod is safely mounted to the pedicle screw 100, a surgeon grips the protrusive rim 651 of the outer sleeve 650 and then moves it in a reverse order to the above. In other words, the surgeon manipulates the outer sleeve 650 such that the guide pin 653 of the outer sleeve 650 is moved from the lower portion to the upper portion of the second vertical sliding guide unit 623c, from a left side to a right side of the horizontal guide unit 623b (based on Fig. 20), and from the lower portion to the upper portion of the first vertical sliding guide unit 623a, thereby releasing the gripping of the pedicle screw 100 and then removing the rod guide 600.

After the surgical operation is completed, in order to separate easily the pedicle screw 100 from the rod guide 600, the minimally invasive spine surgery system 1000 may further include a rod guide separator 400. Figs. 23 to 26 show the rod guide separator 400. The rod guide separator 400 includes a hollow sleeve 410 fixedly connected to the end of the inner body 610 of the rod guide 600 and mounted to two facing grooves formed in the end portion of the fixed surface 630 of the inner body 610 and a T shaped insert bar 420 inserted into the sleeve 410.

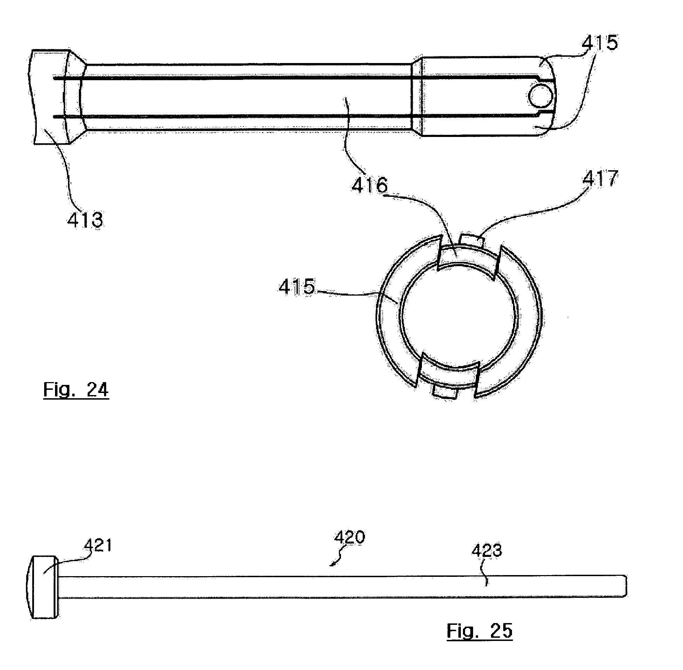

As shown in Fig. 23, the sleeve 410 includes a fixed unit 411 having an opening formed thereon and screw-coupled to the inner body 610, a hollow body 413 extending downward from the fixed unit 411, two rigid arms 415 extending from end of the hollow body 413 to face each other and two flexible arms 416 extending from the end of the hollow body 413 and being adjacent to sides of the rigid arms 415 and facing each other. Also, a protrusion 417 is formed on an outer surface of the flexible arm 416, and the flexible arm 416 is shrunken inward rather than the rigid arm 415 as shown in Fig. 24. A protrusion having a shape complementing to that of the groove 631 of the inner body 610 is formed on the fixed unit 411, so that the protrusion is mounted in the groove 631 of the inner body. Accordingly, the rod guide separator 400 is guided such that the protrusion 417 formed on the flexible arm 416 and the gripping portion 617 of the inner body 610 are located on one straight line. Preferably, outer diameters of the hollow body 413 and the rigid arm 415 are substantially identical to an inner diameter of the inner body 610 of the rod guide 600, and an outer diameter of the flexible arm is smaller than an inner diameter of the inner body 610.

As shown in Fig. 25, the insert bar 420 includes a circular handle 421 and a cylindrical bar 423 extending downward from the center of the circular handle 421 and passing through the hollow body 413. Also, the cylindrical bar has a length greater than the entire length of the sleeve 410. Preferably, the outer diameter of the cylindrical bar 423 is substantially identical to the inner diameters of the hollow body 413 and the rigid arm 415, and the end of the cylindrical bar 423 is tapered.

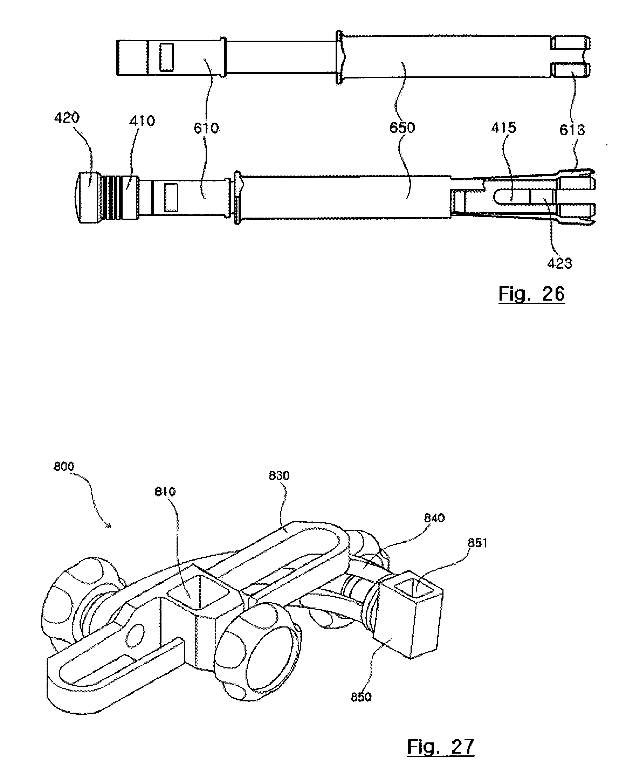

The operating mechanism of the rod guide separator 400 is explained with reference to Fig. 26. First, the fixed unit 411 of the sleeve 410 is mounted to the groove 631 of the inner body 610 of the rod guide 600. At this time, the hollow body 413, the rigid arm 415 and the flexible arm 416 of the sleeve are completely received or inserted into the inner body 610, and the protrusion 417 formed on the flexible arm 416 is located near the flexible arm 613 of the inner body 610. Then, the insert bar 420 is inserted into the sleeve 410. Since the outer diameter of the cylindrical bar 423 of the insert bar 420 is substantially identical to the inner diameter of the rigid arm 415 and greater than the inner diameter of the flexible arm 416, when the insert bar 420 is inserted into the sleeve 410, the end of the circular bar 423 expands the flexible arm 416 outwards. Due to the above, the protrusion 417 formed on the flexible arm 416 of the sleeve 410 expands the flexible arm of the inner body 610 of the rod guide 600 outwards, thereby separating the pedicle screw 100 from the rod guide 600.

By using the above rod guide separator 400, the pedicle screw 100 may be easily separated from the rod guide 600, so that it is possible to prevent any muscle or tissue around a surgical operation region from being damaged during the rod guide separating process.

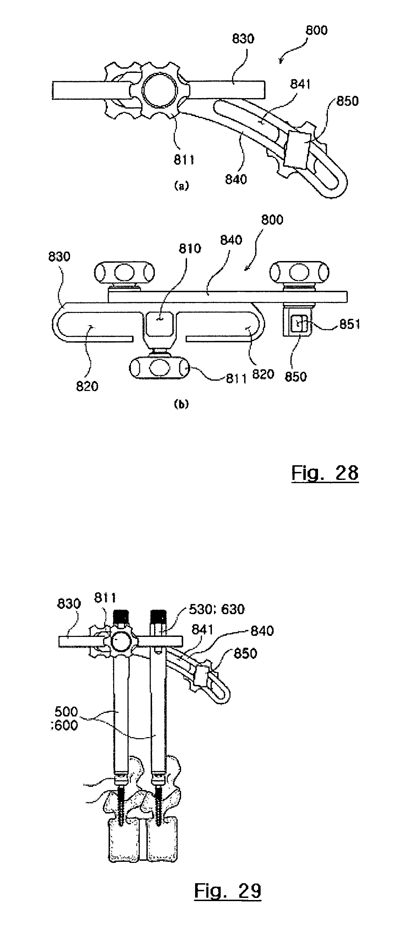

Figs. 27 to 29 are schematic views showing a structure and an installation state of the rod guide holder 800 constituting the minimally invasive spine surgery system 1000 according to the present invention. Fig. 27 is a perspective view of the rod guide holder 800 of the present invention, Figs. 28a and 28b are a side view and a plane view of the rod guide holder 800 of the present invention, and Fig. 29 is a schematic view showing a state where the rod guide holder 800 of the present invention is installed on the rod guides 500, 600.

Referring to Figs. 27 and 28, the rod guide holder 800 includes a first fixing unit 830, a guide 840 and a second fixing unit 850. Here, the first fixing unit has a rectangular first fixture 810 formed at body center thereof for inserting one of the pair of rod guides 500, 600 therein and a U shaped second fixture 820 formed at both sides of the first fixture 810 for inserting the remaining rod guide therein, and the guide 840 is connected to one side of the first fixture 810 of the first fixing unit 830 and has an arc slit 841 formed thereon. Also, the second fixing unit 850 is connected to the guide 840 and rotated and moved along the slit 841 to guide an insert position of the rod in a state where the rod holder 300 is inserted in the second fixing unit.

A knob 811 is formed at one side of the first fixture 810 for fixing the rod guides 500, 600 after the rod guides 500, 600 are inserted therein, and a screw is formed on the knob 811 to enable the rod guides 500, 600 to be fixed or released according to a rotating direction of the knob 811.

Also, the second fixture 820 has a U shape, so that the cylindrical rod guides 500, 600 may be moved along the second fixture 820 as much as a predetermined distance.

Preferably, the inner shape of the first fixture 810 and the inner shape and size of the second fixture 820 are determined such that the planed fixed surfaces 530, 630 formed perpendicularly to one pair of cuts 510, 515 can be mounted at the upper end of the rod guides 500, 600 without rotating. By using the above configuration, it is possible to prevent the rod guides 500, 600 from being rotated in the rod guide holder 800, so that it is fundamentally possible to prevent a location of the cuts 510, 615 serving as a path of the rod from being changed. Fig. 23 illustrates that one rod guide is inserted into the first fixture 810 and fixed by the knob 811, and the remaining rod guide is inserted into the second fixture 820 located at the right side (based on Figs. 27 to 29) such that the rod guide holder 800 is installed to the rod guides 500, 600.

The second fixing unit 850 has an insert hole 851 into which the rod holder 300 may be inserted and is connected such that the second fixing unit can be moved circularly at the slit 841 formed in the guide 840 and rotated freely, so that the rod 200 gripped by the rod holder 300 may be accurately mounted to the pedicle screw 100 while freely adjusting an insert angle into the rod guides 500, 600.

By the above configuration, the rod guides 500, 600 and the rod holder 300 may be located on a straight line, so that it is possible to prevent the rod holder 300 from being separated from the insertion path of the rod 200. In addition, since the path along which the rod holder 300 is moved is placed within a radius of rotation, the rod may be easily inserted. Moreover, during a surgical operation, a scattering of the arrangement of four cuts on the straight line caused by a rotation of rod guides 500, 600 can be prevented due to the contact between the rod holder 300 and the rod guide, thereby improving the accuracy of the rod inserting process.

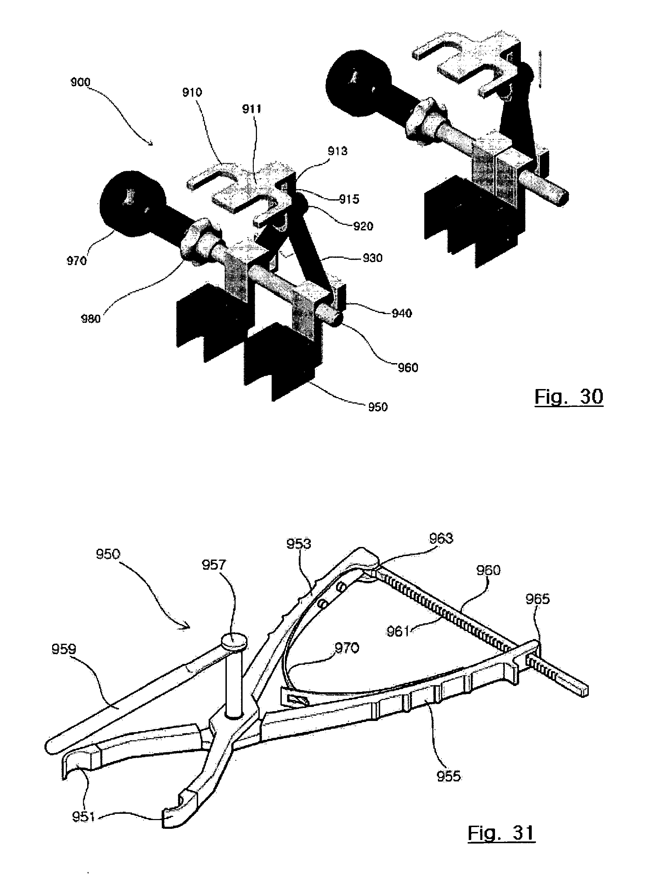

Fig. 30 is a schematic perspective view showing configuration and operating mechanism of the gap adjuster 900 for suitably adjusting a gap between the head portions of the pedicle screw 100.

As shown in Fig. 30, the gap adjuster 900 includes a fixed gripping portion 910, two legs 930 connected to a lower portion of the fixed gripping portion 910 by a hinge 920, arms 940 rotatably connected to the two legs 930, respectively, an adjustment gripping portion 950 fixedly connected to the arm 940, and a bar member 960 having a male thread screw-coupled to a bore formed in the arm 940 and having a female thread formed therein.

The fixed gripping portion 910 includes a horizontal portion 911 and a vertical portion 913 so that the fixed gripping portion has a '¬' shape as a whole when being seen from a side. The horizontal portion 911 has a '3' shape and grips fixedly the plane grooves 521, 621 formed on the upper portions of the pair of rod guides 500, 600, and the vertical portion 913 has a slit 915 formed thereon.

By means of the hinge 920, two legs 930 are connected to the slit 915 formed in the fixed gripping portion 910, so that an angle between two legs 930 may be changed as the two legs 930 are widened or narrowed.

The arms 940 are rotatably connected to ends of the two legs 930, respectively, preferably by pins. A bore on which a female thread is formed is formed on the arm 940.

The bar member 960 having a male thread formed thereof passes through the bore of the arm 940 having the female thread formed therein. At this time, the bar member 960 and the bore are screw-coupled to each other like a bolt and screw. A handle 929 is installed at one end of the bar member 960 to facilitate a rotation of the bar member 960, and a knob 931 is installed at the front of the handle 929. The knob 931 plays a role of fixing the adjusted gap during a surgical operation process after a gap between the pedicle screws 100 is adjusted by turning the handle 929.

The adjustment gripping portion 950 is fixedly and perpendicularly mounted to one end of the arm 940 and grips central sides of the rod guides 500, 600 or a central side of the outer sleeve 650. Preferably, the adjustment gripping portion 950 has shape and size complementing to those of central sides of the rod guides 500, 600 or a central side of the outer sleeve 650, so that the adjustment gripping portion 950 may firmly grip the rod guide or the outer sleeve 650.

Hereinafter, the operating mechanism of the gap adjuster 900 is illustrated.

First of all, the horizontal portion 911 of the fixed gripping portion 910 is located in the plane grooves 521, 621 of the inner body 610, and the adjustment gripping portion 950 is located at the central side of the rod guide 500 or the central side of the outer sleeve 650. In case where a gap between pedicle screws fixed to the vertebral pedicle is wider than a required value, the handle 929 is rotated to move the bar member 960 to a position opposite to the handle 929. Due to the above movement of the handle 929 in the opposite direction, two arms 940 become closer, so that the distance between the adjustment gripping portions 950 fixed to two arms 940 is also decreased. Thus, the distance between the head portions of the pedicle screws 100 gripped by the rod guides 500, 600 can be decreased. When the gap of the adjustment gripping portion 950 is decreased as above, the angle between the legs 930 is decreased, and the hinge 920 connecting the legs 930 is moves upward along the slit 915 formed on the vertical portion 913 of the fixed gripping portion 910.

On the contrary, in case where the gap between the pedicle screws fixed to the vertebral pedicle is narrower than a required value, by rotating the bar member 960 in a direction opposite to the case where that the gap between the pedicle screws fixed to the vertebral pedicles is wider than a required value, the gap between the pedicle screws may be widened in the same principle as described above.

By using the gap adjuster 900 configured as above, it is possible to solve an inconvenience caused by manipulating directly the long rod guides 500, 600 by hand to adjust a gap between the pedicle screws 100 and a problem that the force is not accurately transferred to the screw portion of the pedicle screw 100 due to such a manual manipulation. In other words, by means of the gap adjuster 900, a gap between the pedicle screws 100 may be decreased just by determining a rotating direction of the handle 929, and it is possible to easily adjust a gap of the pedicle screws 100 by rotating the handle 929 with a small force.

As another embodiment of the gap adjuster, as shown in Figs. 31 to 33, the gap adjuster 950 may comprise a first handle 953 and a second handle 955 respectively having adjustment gripping portions 951 mounted to one ends thereof, a T shaped hinge shaft 957 connecting the first handle 953 and the second handle 955 in a hinge manner and mounted vertically at connection portions of the first handle 953 and the second handle 955, and a cylindrical bar 959 mounted to one end of the hinge shaft 957 such that the cylindrical bar can be rotated with respect to the hinge shaft 957.

Preferably, the adjustment gripping portions 951 are rotatably mounted to the first handle 953 and the second handle 955. By mounting rotatably the adjustment gripping portions 951 as mentioned above, although a gap between the adjustment gripping portions 951 is changed by pressing the first handle 953 and the second handle 955, a contact between the adjustment gripping portions 951 and the rod guide can be maintained (in other words, a contact area between the adjustment gripping portion 951 and the rod guide may be maintained) , so that the gap between the pedicle screws may be stably adjusted without a slip of the adjustment gripping portion 951.

Preferably, a bar shaped distance adjusting means 960 having toothed first projections 961 formed on one side thereof is additionally provided between the other end of the first handle 953 and the other end of the second handle 955 to adjust a moving distance of the adjustment gripping portions 951. An insertion hole 965 having a toothed second projection formed at a portion to be in contact with one of concave portions between the first projections 961 is formed with a predetermined depth on the other end of the second handle 955 to enable the distance adjusting means 960 to be inserted therein.

Also, one end of the distance adjusting means 960 is connected to the other end of the first handle 953 by means of a hinge 963, and the other end of the distance adjusting means 960 is inserted into the insertion hole 965 such that one of concave portions between the first projections 961 and the second projection are engaged with each other, so that if the first handle 953 and the second handle 955 are pressed, a moving distance of the adjustment gripping portions 951 may be adjusted step by step.

Preferably, the gap adjuster 950 further includes an elastic member 970 provided between the first handle 953 and the second handle 955 to restore the first handle 953 and the second handle 955 to an original state before being pressed. More preferably, the elastic member 970 consists of two flexible metal plates, but not limited thereto.

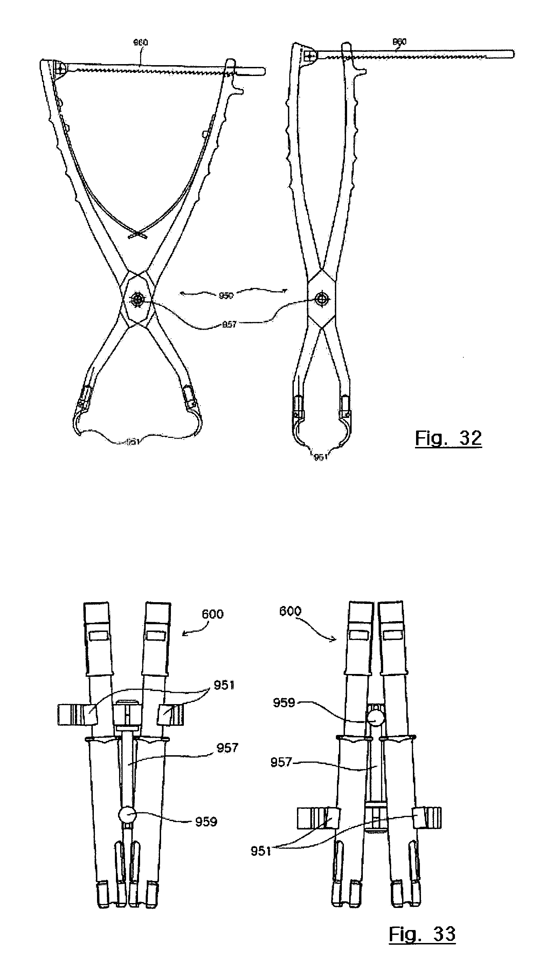

Figs. 32 and 33 show an operating mechanism of the gap adjuster 950. The adjustment gripping portions 951 of the first handle 953 and the second handle 955 are located at outer sides of two rod guides 500, 600 fixed to the vertebra, and the cylindrical bar 959 is inserted between the two rod guides 500, 600. At this time, in order to decrease a gap between the pedicle screws 100, the cylindrical bar 959 is located above the rod guides 500, 600, and the adjustment gripping portions 951 are located below the rod guides. Meanwhile, in order to increase a gap between the pedicle screws 100, the cylindrical bar 959 is located below the rod guides 500, 600, and the adjustment gripping portions 951 are located above the rod guides. Then, the first handle 953 and the second handle 955 are pressed to adjust a gap between the pedicle screws 100. At this time, the gap adjuster is operated in a lever principle, and the hinge shaft plays a role of a support of the lever.

By using the gap adjuster 950 according to the above embodiment, a gap between the pedicle screws 10 may be adjusted more easily with a small force.

The rod holder according to the present invention has an advantage that the rod can be securely gripped and easily inserted and mounted at the same time.

Further, the rod holder according to the present invention can be easily manipulated.

Accordingly, the rod holder according to the present invention can shorten a time required for spine surgical operation.

In addition, according to the present invention, the rod can be placed on a pedicle screw in a more accurate and stable way, thereby minimizing a damage of nerves and tissues around a surgical site when the rod is inserted.

Due to the above effects, the time required for recovering a surgical site of a patient may be shortened so that additional costs after the surgical operation can be reduced.

The preferred embodiments of the present invention have been illustrated and explained herein, but the scope of the present invention is not limited to the embodiment described and illustrated herein, but is defined by the appended claims. It will be apparent that those skilled in the art can make various modifications and changes thereto within the scope of the invention defined by the claims. Therefore, the true scope of the present invention should be defined by the technical spirit of the appended claims.

## Claims

1. A rod holder for gripping a rod (200), which connects a pair of pedicle screws (100) inserted into and fixed to a vertebra with each other, to mount the rod (200) to the pair of pedicle screws (100), the rod holder comprising:
a grip (310) serving as a handle;
a loading unit (350) mounted to an upper portion of the grip (310) in such a manner that the loading unit passes through the grip (310) in a front and rear direction;
a button unit (370) mounted to the upper portion of the grip (310) in such a manner that the button unit passes through the grip (310) in a right and left direction, thereby cooperating with the loading unit (350); and
an insert unit (330) partially inserted into a human body and comprising a rod gripping portion (331) for gripping the rod (200) comprising two arms (331b) configured in a way that the two arms (331b) are elastically divided into a Y shape to adjust a gripped state of the rod (200), and a connection member (333) for transmitting a forward or backward movement of the loading unit (350) to the rod gripping portion (331),
wherein the rod holder is configured to adjust a gripped state of the rod (200) through three sequential stages including a first loading stage for moving backward and fixing the loading unit (350) to fixedly grip the rod (200); a second loading stage for partially moving the loading unit (350) forward and fixing the loading unit (350) to the button unit (370), thereby rotatably gripping the rod (200); and a rod mounting stage for completely moving the loading unit (350) forward by operating the button unit (370) so that the rod (200) is separated from the rod holder (300).

2. The rod holder as claimed in claim 1,
wherein the loading unit (350) includes an intermediate member (351) fixedly connected to a rear end of the connection member (333) and inserted into a rear end (335a) of the insert unit (330) to be elastically movable in a forward or rearward direction; a two-stage loading adjustment member (353) connected to a rear end of the intermediate member (351); and a loading handle (355) fixedly connected to a rear end (353c) of the two-stage loading adjustment member (353),
wherein the intermediate member (351) has a protrusive rim (351a) formed at a front end thereof, and the protrusive rim (351a) is equipped with a spring (337),
wherein the two-stage loading adjustment member (353) has a first catching step (353d) formed at an upper portion thereof for forming the first loading stage and a second catching step (353e) formed at a lower portion thereof for forming the second loading stage at a front side of the first catching step (353d), and
wherein the rod holder further comprises a cover unit (313) provided at an rear portion of an upper side of the grip (310) through which the loading unit (350) passes, the cover unit being formed with a stop to which the first catching step (353d) is fixed in the first loading stage.

3. The rod holder as claimed in claim 1,
wherein the insert unit (330) further comprises a tube shaped hollow insert body (335) into which the rod gripping portion (331) or the connection member (333) is inserted, the hollow insert body (335) forming a forward or backward movement path for the rod gripping portion (331) or the connection member (333),
wherein the insert body (335) comprises a rod gripping portion receiver (335c) into which the rod gripping portion (331) is inserted,
wherein the rod gripping portion receiver (335c) has an internal shape to press the two arms (331b) of the rod gripping portion (331),
wherein the button unit (370) includes a housing (371) passing through the grip (310) in a right and left direction to be mounted to an upper inner portion of the grip (310); a second loading stage adjuster (373) elastically reciprocating in the housing (371); and a button (375) fixed at an end of the second loading stage adjuster (373), and
wherein the second loading stage adjuster (373) has a step portion (373e) formed at an upper portion thereof to adjust the fixing or forward movement of the second catching step (353e) formed at a lower portion of the two-stage loading adjustment member (353) of the loading unit (350).

4. The rod holder as claimed in claim 1,
further comprising a locking unit (390), which includes a circular body (391) located below the two-stage loading adjustment member (353) of the loading unit (350); a wing shaped locking projection (393) extending outward in a radial direction from one side of the circular body (391) to press the two-stage loading adjustment member (353) upward; a bar shaped arm (397) extending outward in a radial direction at the other side of the circular body (391) and having a handle pin (395) mounted thereto; and a central rotary shaft (399) inserted into a through opening (398) formed at a center of the circular body (391) to rotatably fix the circular body (391) to the grip (310).

5. A minimally invasive spine surgery system for inserting and fixing a pair of pedicle screws (100) into and to a vertebra and installing a rod (200) for connecting the pair of pedicle screws (100) with each other, the system comprising:
a pair of rod guides (500, 600) connected to upper ends of the pair of pedicle screws (100) to form a moving path of the rod (200);
a rod holder (300) according to any one of claims 1 to 4 for gripping the rod (200); and
a rod guide holder (800) defining an insertion path of the rod holder (300) to guide an insert position of the rod (200),
wherein the rod guide holder (800) includes a first fixing unit (830) connected to upper ends of the pair of rod guides (500, 600) to keep a distance between the pair of rod guides (500, 600) constant; a guide (840) connected to a center of the first fixing unit (830) and having an arc shaped slit (841) formed thereon; and a second fixing unit (850) connected to the guide (840) and moving circularly along the slit (841) in a state where the rod holder (300) is inserted therein to thereby guide an insert position of the rod (200),
wherein the rod guide holder (800) has a knob (811) formed at a center of the first fixing unit (830) to fix one of the pair of rod guides (500, 600), and
wherein the second fixing unit (850) is rotatably connected to the slit (841) so that an angle at which the rod (200) gripped by the rod holder (300) is inserted into the rod guides (500, 600) may be freely adjusted.

6. The minimally invasive spine surgery system as claimed in claim 5,
wherein each of pair of rod guides (600) includes a hollow cylindrical inner body (610) for gripping the pedicle screw (100); and an outer sleeve (650) arranged around the inner body (610) in an axial direction and slid in two stages along the inner body (610),
wherein the outer sleeve (650) has a protrusive rim (651) formed at an upper end thereof,
wherein the outer sleeve (650) has a pair of cuts (655) formed at a lower end thereof in opposite directions so that the rod (200) can be inserted into the cuts,
wherein the inner body (610) has a pair of cuts (615) formed at a lower end thereof in opposite directions to allow the rod (200) to be inserted in the cuts and a plurality of elastic arms (613) formed at a lower end thereof and widen into a V shape to elastically adjust a gripped state of the pedicle screw (100), and
wherein at least one rod pressing unit (657) extends downward from a lower end of the outer sleeve (650), and the rod pressing unit (657) presses an upper surface of the rod so that the rod (200) is closely contacted with a head portion (110) of the pedicle screw (100).

7. The minimally invasive spine surgery system as claimed in claim 6,
wherein the inner body (610) has a pair of plane grooves (621) symmetrically formed on an upper surface thereof, and the plane groove (621) and the cut of the inner body (610) are located on a straight line,
wherein the system further comprises a rod pusher (700), which includes an upper handle (720) having a fixed gripping portion (710) for gripping the groove of the inner body (610), and a lower handle (740) having a movable gripping portion (747) pressing the protrusive rim (651) of the outer sleeve (650) toward the pedicle screw and connected to the upper handle (720) through a hinge (730), and
wherein the rod pusher (700) grips the plane groove (621) of the inner body (610) and the protrusive rim (651) of the outer sleeve (650) to press the outer sleeve (650) toward the pedicle screw (100).

8. The minimally invasive spine surgery system as claimed in claim 6,
wherein the system further comprises a rod guide separator (400), which includes a hollow sleeve (410) fixedly connected to an end of the inner body (610) of the rod guide (600) and inserted into the inner body (610), and a T shaped insert bar (420) inserted into the sleeve (410), and
wherein the sleeve (410) includes a fixed unit (411) having an opening mounted to the groove (631) of the inner body (610), a hollow body (413) extending downward from the fixed unit (411), two rigid arms (415) extending from an end of the hollow body (413) and facing each other, and two flexible arms (416) extending from the end of the hollow body (413) and facing each other adjacent to a side of the rigid arms (415), wherein a protrusion (417) is formed at an outer surface of the flexible arms (416), and the flexible arms (416) are shrunken more inward than the rigid arms (415), wherein the insert rod (420) includes a circular handle (421) and a cylindrical bar (423) extending downward from a center of the circular handle (421), passing through the hollow body (413) and having a length longer than an entire length of the sleeve (410).

9. The minimally invasive spine surgery system as claimed in claim 5,
wherein the system further comprises a gap adjuster (900), which includes a fixed gripping portion (910) for gripping upper portions of the rod guides (500, 600), two legs (930) connected to the fixed gripping portion (910) through a hinge (920), arms (940) rotatably connected to the two legs (930), respectively, an adjustment gripping portion (950) fixedly connected to the arms (940), and a bar member (960) having a male thread formed thereon and screw-coupled with a bore having a female thread formed in the arms (940), and
wherein the gap adjuster (900) can adjust a gap between the pedicle screws (100) by rotating the bar member (960).

10. The minimally invasive spine surgery system as claimed in claim 5,
wherein the system further comprises a gap adjuster (950), which includes a first handle (953) and a second handle (955) respectively having adjustment gripping portions (951) rotatably mounted to one ends thereof, a T shaped hinge shaft (957) connecting the first handle (953) and the second handle (955) in a hinge manner and mounted perpendicularly at a connection portion of the first handle (953) and the second handle (955), and a cylindrical bar (959) mounted to one end of the hinge shaft (957) to be rotatable about the hinge shaft (957).
